# EUROPÄISCHE PATENTANMELDUNG

(11) **EP 1 600 164 A2**
(43) Veröffentlichungstag der Anmeldung: **30.11.2005**
(21) Anmeldenummer: 04090337.9
(22) Anmeldetag: 01.09.2004
(51) Int. Cl.: A61K 39/395

(54) **Verwendung einer an CD28 bindenden Wirksubstanz zur Herstellung einer Pharmazeutischen Zusammensetzung mit dosisabhängiger Wirkung**

(30) Priorität: 22.09.2003 DE 10345008; 20.10.2003 DE 10349371
(71) Anmelder: TeGenero AG, 97076 Würzburg (DE)
(72) Erfinder: Hanke, Thomas, Dr., 97078 Wuerzburg (DE); Lin, Chia-Huey, 97078 Wuerzburg (DE)
(74) Vertreter: Jungblut, Bernhard Jakob

(57) **Zusammenfassung**

Die Erfindung betrifft die Verwendung eines CD28-spezifischen superagonistischen monoklonalen Antikörpers (mAb) oder einer Mimikriverbindung hierzu, zur Herstellung einer pharmazeutischen Zusammensetzung, wobei die Dosis unterhalb oder oberhalb einer definierten Grenzdosis liegt.

## Beschreibung

### Gebiet der Erfindung.

Die Erfindung betrifft die Verwendung einer an CD28 bindenden Wirksubstanz zur Herstellung einer pharmazeutischen Zusammensetzung.

### Hintergrund der Erfindung und Stand der Technik.

Autoimmunität entsteht, wenn das adaptive Immunsystem bestehend aus B- und T-Lymphozyten (B- und T-Zellen) körpereigene Strukturen ("selbst") für körperfremd hält und seine Effektormechanismen gegen diese mobilisiert. Multiple Sklerose, Typ 1 Diabetes und Rheumatoide Arthritis (RA) sind Beispiele von Autoimmunerkrankungen, bei denen angenommen wird, dass im Körper vorhandene selbst-reaktive T-Lymphozyten eine zentrale Rolle bei der Pathogenese der Erkrankung spielen (Ermann and Fathman, 2001).

In Normalfall sind T-Zellen tolerant gegenüber körpereigenem Gewebe und reagieren nur auf die Haupt-Histokompatibilitätskomplex (MHC) vermittelte Erkennung körperfremder Strukturen mit Aktivierung und Effektorfunktionen. Dies ist auf verschiedene"Toleranz-induzierende" Mechanismen zurückzuführen: 1) Negative Selektion: Während ihrer Reifung im Thymus werden solche T-Zellen eliminiert, deren Antigenrezeptoren eine hohe Affinität für selbst-Antigene besitzen und damit potentiell autoaggressiv und pathogen sind; 2) Anergie-Induktion: Bei einer unvollständigen Stimulation von T-Zellen, z.B. durch fehlende Kostimulation in sekundären lymphoiden Organen, können autoreaktive T-Zellen in einen Zustand der zeitweiligen funktionellen Passivität oder Anergie versetzt werden; 3) Aktive Suppression: Autoreaktive und potentiell pathogene T-Zellen werden durch eine spezialisierte T-Zell-Subpopulation, sogenannte regulatorische T-Zellen (Treg, früher auch als "Suppressorzellen" bezeichnet), aktiv an der Ausübung von Effektorfunkionen gehindert.

Die große Bedeutung dieses Mechanismen für die Aufrechterhaltung immunologischer Toleranz ist in zahlreichen aktuellen Übersichtsartikeln zusammengefasst (Maloy and Powrie, 2001; Sakaguchi et al., 2001; Shevach, 2002).

Die wesentlichen Charakteristika der regulatorischen T-Zellen lassen sich wie folgt beschreiben: i) Es handelt sich um CD4 T-Lymphozyten mit einem breiten Repertoire an T-Zell-Antigenrezeptoren (TCR) des konventionellen α/β Typs. Regulatorische T-Lymphozyten reifen im Thymus und sind in den peripheren lymphoiden Organen gemeinhin durch Expression der niedermolekularen Isoform des CD45 Moleküls, der Rezeptoren CD25 (IL-2R alpha Kette), CD152 (CTLA-4), Glucocorticoid Induced TNF Receptor Family Related Protein (GITR) und des Transkriptionsfaktors foxp3 (Khattri et al., 2003) gekennzeichnet. Durch kombinierte Analyse dieser Marker gelingt eine weitgehende Abgrenzung regulatorischer T-Zellen vom Rest der CD4 T-Zellpopulation.

Durch Elimination regulatorischer T-Lymphozyten *in vivo* einerseits und adoptive Zelltransferexperimente andererseits konnte gezeigt werden, dass regulatorische T-Lymphozyten für die Verhinderung organspezifischer autoimmunentzündlicher Erkrankungen wie der Colitis, der Insulitis oder der Orchitis wichtig sind. Dabei scheint das zahlenmäßige Verhältnis von regulatorischen T-Zellen und konventionellen T Zellen, die durch das Fehlen von Treg zu pathogenen TZellen werden können, eine wichtige Rolle zu spielen (Sakaguchi et al., 1995).

Der Wirkmechanismus von regulatorischen T-Zellen ist noch nicht vollständig aufgeklärt. *In vivo*, nicht aber in Zellkultur, scheinen die anti-inflammatorischen Zytokine IL-10 und TGFβ eine wichtige Rolle zu spielen (Maloy and Powrie, 2001). Aus Zellkulturexperimenten geht andererseits klar hervor, dass direkter Kontakt zwischen den regulatorischen und den zu supprimierenden T-Lymphozyten essentiell ist; möglicherweise kommt CTLA-4 hierbei eine funktionelle Bedeutung zu (Thornton and Shevach, 1998). Auch ohne die noch nicht eindeutig geklärte Wirkungsweise regulatorischer T-Lymphozyten zeichnet sich deutlich ab, dass hier ein allgemeines Prinzip der Aufrechterhaltung der immunologischen Selbsttoleranz vorliegt, das für die gezielte Bekämpfung von Autoimmunität nutzbar gemacht werden kann.

Ziel einer pharmakologischen Aktivierung regulatorischer T-Zellen für die Therapie von Autoimmunerkrankungen ist, die Vermehrung und Funktion dieser Zellen im Organismus anzuregen, ohne dass die Zellen ihre suppressive Funktion verlieren. Dieser Ansatz hat sich bislang experimentell als äußerst schwierig erwiesen. So führt die Aktivierung von regulatorischen T-Zellen, folgend auch Treg genannt, über den TCR auch in Kombination mit einem konventionellen Signal über das kostimulatorische Molekül CD28 in der Regel nicht zu einer Proliferation dieser Zellen.

Im Tiermodell Ratte konnte gezeigt werden, dass ein neuartiger monoklonaler Antikörper (MAB) mit Spezifität für das CD28 Molekül -JJ316- T-Lymphozyten sowohl in vitro als auch in vivo ohne TCR Stimulation effizient aktiviert (Tacke et al., 1997), also "superagonistisch" wirkt. Dieser Antikörper wird trotz seiner starken T-Zell-stimulatorischen Eigenschaften im Gegensatz zu allen anderen bekannten T-Zell-aktiviernden Substanzen in vivo bestens toleriert (Rodriguez-Palmero et al., 1999; Tacke et al., 1997). Neueste Ergebnisse zeigen, dass bei in vivo Gabe von JJ316 funktionelle Treg in der Ratte überproportional stark expandiert werden (Lin and Hunig, 2003). Diese präferentielle Stimulation regulatorischer T Zellen mit einem CD4+CD25+CTLA-4+ Phänotyp ist höchstwahrscheinlich der Grund für die gute Verträglichkeit und die entzündungshemmende Wirkung des Antikörpers im Organismus (siehe auch DE 197 22 888; PCT/DE03/00890).

Als neuen Ansatz zur Vermehrung von Treg für therapeutische Zwecke im Menschen ist ein superagonistischer Antikörper mit Spezifität für das humane CD28-Molekül des Menschen, genannt CD28-SuperMAB, entwickelt worden. Diese mAk regen in vitro T-Lymphozyten des Menschen sehr effizient zur Zellteilung an (Luhder et al., 2003). Erste Untersuchungen sprechen dafür, dass sich ähnlich wie bei der Ratte die im peripheren Blut des Menschen in geringer Zahl vorhandenen CD4+CD25+ regulatorischen T-Zellen zwar schlecht durch Kostimulation, d.h. durch anti-TCR plus konventionelle anti-CD28 Stimulation, aber sehr gut durch superagonistische CD28 Stimulation vermehren lassen (PCT/DE03/00890). Darüber hinaus konnte in einem Pilotversuch gezeigt werden, dass eine in vivo Gabe von anti-human CD28-SuperMAB bei einem Rhesusaffen in vivo eine profunde Aktivierung von T-Zellen induziert, ohne dass es zu klinisch sichbaren Nebenwirkungen kommt. Dies spricht dafür, dass auch der human CD28-SuperMAB eine anti-phlogistische Wirkung haben könnte. Zusammenfassend zeigten diese Versuche, dass der CD28-SuperMAB in vitro humane Treg aktiviert und in vivo in einem Tiermodell trotz Induktion einer T-) Zell-Aktiverung sehr gut verträglich ist.

### Vorstehend zitierte Literatur:

Barnes, D. A., et al., (1998) J Clin Invest 101, 2910-2919.
Ermann, J., et al., (2001) Nat Immunol 2, 759-761.
Khattri, R., et al., (2003) Nat Immunol 4, 337-342.
Lin, C. H., et al., (2003) Eur J lmmunol 33, 626-638.
Luhder, F., et al., (2003) J Exp Med 197, 955-966.
Maloy, K. J., et al., (2001) Nat Immunoi 2, 816-822.
Rodriguez-Palmero, M., et al., (1999) Eur J Immunol 29, 3914-3924.
Sakaguchi, S., et al., (1995) J Immunol 155, 1151-1164.
Sakaguchi, S., et al., (2001). Immunol Rev 182, 18-32.
Shevach, E. M. (2002) Nat Rev lmmunol 2, 389-400.
Tacke, M., et al., (1997) Eur J Immunol 27, 239-247.
Thornton, A. M., et al., (1998) J Exp Med 188, 287-296.

### Technisches Problem der Erfindung.

Der Erfindung liegt das technische Problem zu Grunde, eine pharmazeutische Zusammensetzung anzugeben, welche sowohl als endzündingshemmendes als auch als immunrekonstituierendes Mittel einsetzbar ist.

### Grundzüge der Erfindung und bevorzugte Ausführungsformen.

Zur Lösung dieses technischen Problems lehrt die Erfindung die Verwendung eines CD28-spezifischen superagonistischen monoklonalen Antikörpers (mAb) oder einer Mimikriverbindung hierzu, zur Herstellung einer pharmazeutischen Zusammensetzung zur Behandlung oder Prophylaxe von autoimmunbedingten entzündlichen Erkrankungen oder zur Immunrekonstitution, wobei die pharmazeutische Zusammensetzung mit der Maßgabe hergerichtet ist, dass die darzureichende Dosis des mAb unterhalb einer definierten ersten Grenzdosis liegt, wenn Behandlung oder Prophylaxe einer autoimmunbedingten entzündlichen Erkrankung indiziert ist, und dass die darzureichende Dosis des mAb oberhalb einer definierten zweiten Grenzdosis liegt, wenn Immunrekonstitution indiziert ist.

Die Erfindung beruht auf einer Reihe von neuen Experimenten und daraus gewonnen Erkenntnissen. Solche Experimente haben gezeigt, dass im Rattenmodell niedrige Dosen von JJ316 (superagonistischer CD28 spezifischer mAb) Treg stärker expandieren als konventionelle T Zellen. Zudem zeigt eine niedrige Dosis in einem Modell für Rheumatoide Arthritis die gleiche therapeutische Wirkung wie eine 5-fach höhere Dosis. Die Grundkonzeption der Erfindung besteht somit darin, den entzündungshemmenden Effekt des Antikörpers bei niedriger Dosis von dem allgemein T-Zell-stimulierenden / immunrekonstituierenden Effekt bei hoher Dosis trennen zu können. Mit anderen Worten ausgedrückt, wird eine pharmazeutische Zusammensetzung wahlweise mit einer Dosierung der Darreichungseinheiten und/oder einer Dosierungsanweisung hergerichtet, die je nach gewünschter Indikation (entzündungshemmend / immunrekonstituierend) unterhalb oder oberhalb der respektiven definierten Grenzdosen liegt.

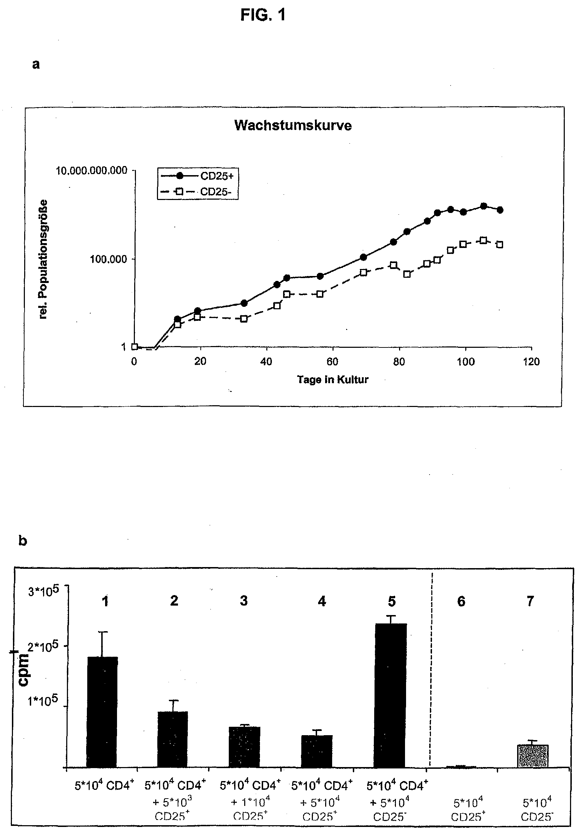

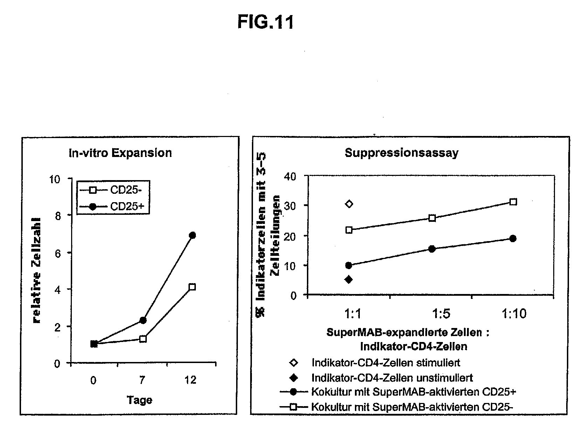

Es wurde als Aspekt von selbstständiger Bedeutung gefunden, dass ein erfindungsgemäßer superagonistischer anti-Ratten- bzw. anti-human CD28 Antikörper eine Langzeitkultur von funktionellen Treg in vitro erlaubt (siehe auch FIG. 1 und 11) und dass daraus die Möglichkeit einer selektiven Expansion von Treg in vitro und einer therapeutischen Re-infusion zur Unterdrückung von Autoimmunerkrankungen erwächst. Insofern ist auch Gegenstand der Erfindung ein Verfahren zur in vitro Expansion von Treg, wobei Treg mit erfindungsgemäßen mAb inkubiert und zumindest über einen Zeitraum von 3 bis 10 Tagen expandiert werden, und zwar vorzugsweise um zumindest den Faktor 2 bis 4 (Zahl der Treg nach der Expansion zur Zahl der Treg vor der Expansion).

In gesunden Ratten ist bei niedrigdosierter Gabe von JJ316 (0,1-0,3 mg/Tier) nach 3 Tagen eine stärkere Expansion von regulatorischen CD4+CD25+ T Zellen in Lymphknoten und Milz zu finden als von konventionellen CD4+CD25- T-Zellen (siehe auch FIG. 2). Diese regulatorischen T Zellen sind phänotypisch aktiviert (FIG. 3) und ein hoher Anteil von Ihnen befindet sich im Zellzyklus (FIG. 4, 5). Die suppressiven Eigenschaften der Treg, gemessen in einem Surrogat-Assay in vitro, sind nach Gabe einer niedrigen Dosis besser als ohne Vorbehandlung (FIG.6).

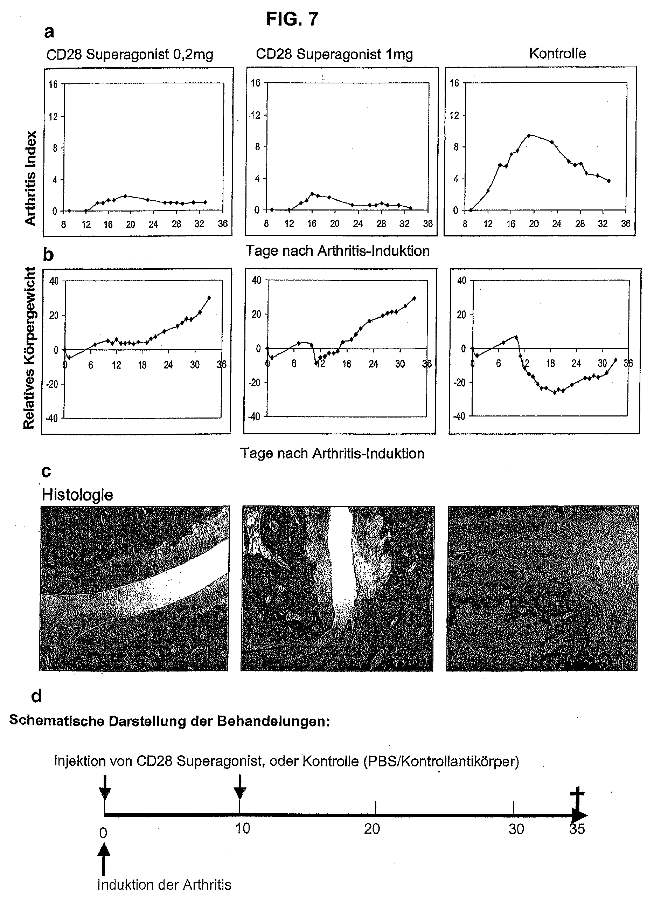

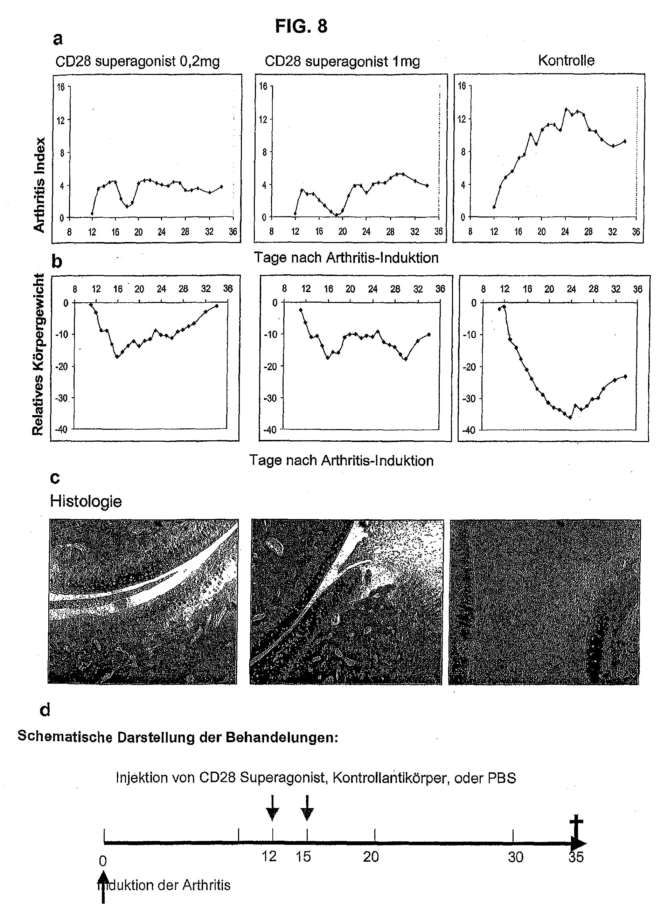

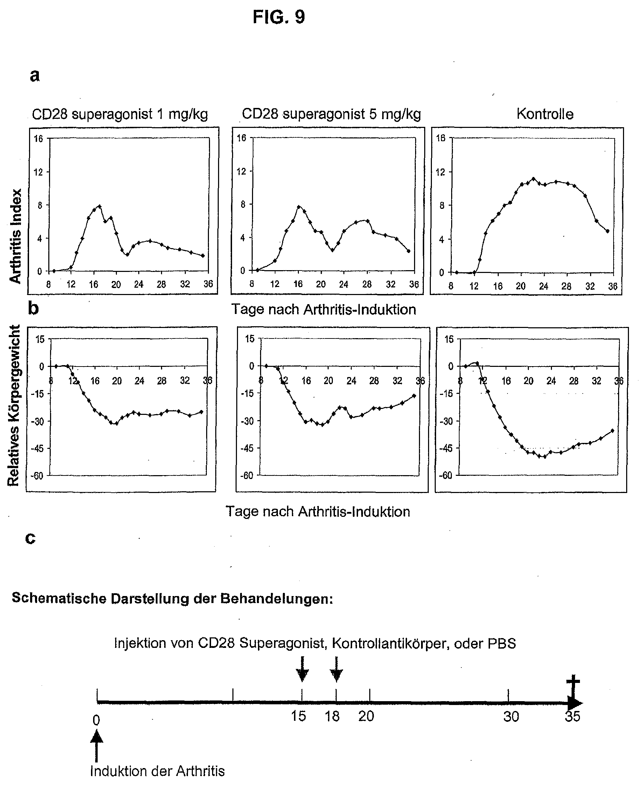

In der vorangegangen Patentanmeldung DE 197 22 888.7 - 41 der Anmelderin wurden die prophylaktischen Eigenschaften des Antikörpers JJ316 bei der Prävention der Ratten-Adjuvans-Arthritis (als Tiermodell für die menschliche RA) angesprochen. In der Zwischenzeit wurden umfangreichere und bessere Daten erhoben, die zeigen, dass sich dieser Effekt nicht nur mit mehr Tieren und besseren Kontrollen wiederholen lässt (FIG. 7), sondern dass Antikörpergabe auch bei einer bereits etablierten Erkrankung therapeutisch wirksam ist (FIG. 8, 9).

Sämtliche vorhergehenden Experimente zu anti-phlogistischen Eigenschaften des Ratten CD28-Superagonisten in den Tiermodellen Adjuvans-Arthritis (DE 197 22 888) und EAN (Patentanmeidung PCT/DE03/00890) waren routinemäßig mit einer - wie sich herausgestellt hat - relativ hohen Antikörperdosis, nämlich 1 mg/Tier, durchgeführt. Die neuen Experimente zeigen, dass zumindest in der Adjuvans-Arthritis auch eine 5-fach niedrigere Dosis (0,2 mg/Tier) den gleichen präventiven bzw. therapeutischen Effekt hat (FIG. 7-9).

Der Umstand, dass Treg bevorzugt bei der niedrigen Dosis expandiert werden während eine hohe Dosis sowohl Treg als auch "normale" T Zellen expandiert, hat die Implikation, dass man die Indikationsfelder "entzündliche Autoimmunerkrankungen" (Wirkmechanismus des Antikörpers vermutlich über die Aktivierung von Treg) und "Immunrekonstitution", d.h. Vermehrung aller T Zellen, durch eine geeignete Dosisfindung trennen kann, i.e. Entzündunghemmung bei niedriger und verträgliche Immunstimulation bei hoher Dosis.

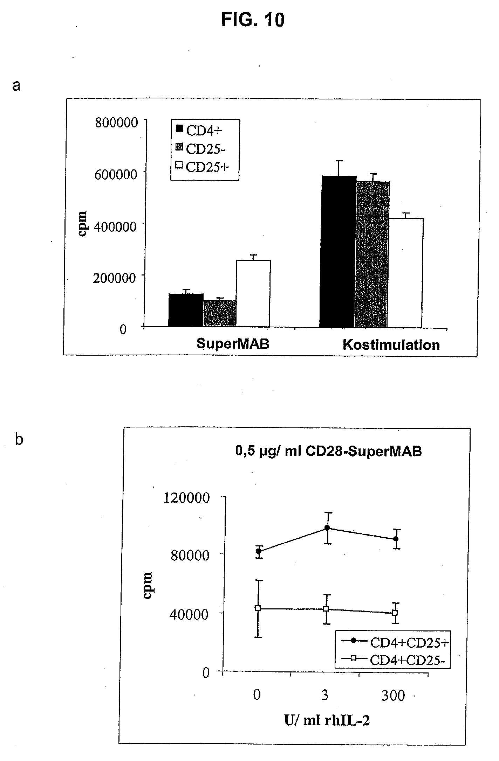

Die Ergebnisse der FIG.10 unterstützen ältere Daten aus der Patentanmeldung PCT/DE03/00890, dass der humane Superagonist in vitro Treg zur Proliferation treiben kann; FIG. 11 zeigt erstmalig, dass die in vitro expandierten CD4+CD25+ Zellen auch suppressorischen Funktion in einem in vitro Assay besitzen.

Bevorzugt ist es, wenn der mAb herstellbar ist, indem ein nichtmenschliches Säugetier mit CD28 oder einer Teilsequenz hieraus, insbesondere dem C'-D Loop und/oder räumlich angrenzenden Bereichen, immunisiert wird, wobei aus dem nichtmenschlichen Säugetier Zellen entnommen und aus den Zellen Hybridomzellen hergestellt werden, und wobei die so erhaltenen Hybridomzellen mit der Maßgabe selektiert werden, daß in deren Kulturüberstand mAb enthalten sind, die an CD28 superagonistisch binden. Aber auch andere übliche Methoden des Erhaltes entsprechender mAb, wie beispielsweise Phage Display oder Human-Ig transgene Mäuse, sind einsetzbar.

Grundsätzlich kann es sich bei erfindungsgemäßen Antikörpern auch um aus den beschriebenen Antikörpern abgeleitete Antikörper handeln, wie beispielsweise um chimäre oder humanisierte Antikörper.

Die Mimikriverbindung ist beispielsweise erhältlich in einem Screeningverfahren, wobei eine prospektive Mimikriverbindung oder eine Mischung von prospektiven Mimikriverbindungen einem Bindungsassay mit CD28 oder einer Teilsequenz hieraus, insbesondere an dem C'-D-Loop und/oder räumlich angrenzenden Bereichen, unterworfen werden, und wobei an CD28 oder an die Teilsequenz hieraus bindende Wirksubstanzen selektiert werden, ggf. gefolgt von einem Assay zur Prüfung auf superagonistische Stimulation von mehreren bis allen Untergruppen der T-Lymphozyten, wobei superagonistisch stimulierende Wirksubstanzen selektiert werden.

Der mAb ist beispielsweise erhältlich aus Hybridomzellen, wie hinterlegt unter den DSM Nummern DSM ACC2531 (mAb: 9D7 bzw. 9D7G3H11) oder DSM ACC2530 (mAb: 5.11A bzw. 5.11A1C2H3). Der mAb oder die Mimikriverbindung enthält vorzugsweise eine oder mehrere der Sequenzen Seq.-ID 9, 11, 13 und/oder 15, oder eine oder mehrere Sequenzen Seq.-ID 10, 12, 14 und/oder 16, oder eine oder mehrere Sequenzen Seq.-ID 18 und/oder 19, oder hierzu homologe Sequenzen.

Die Erfindung betrifft auch ein Verfahren bzw. einen Behandlungsplan zur Behandlung oder Prophylaxe einer vorstehend genannten Erkrankung, wobei entweder einem Patienten eine pharmazeutische Zusammensetzung enthaltend einen CD28-spezifischen superagonistischen monoklonalen Antikörper oder eine Mimikriverbindung hierzu und galenisch hergerichtet für eine definierte und angewandte Darreichungsform, beispielsweise i.v. Injektion, verabreicht wird, oder einem Patienten eine Körperflüssigkeit, insbesondere Blut, enthaltend T-Lymphozyten oder Vorläuferzellen hierzu entnommen wird, die Körperflüssigkeit, ggf. nach einer Aufbereitungsverfahrensstufe, mit einem CD28-spezifischen superagonistischen monoklonalen Antikörper oder einer Mimikriverbindung hierzu versetzt wird und die so behandelte Körperflüssigkeit dem Patienten wieder dargereicht, beispielsweise i.v. injiziert, wird.

Die dargereichte Tagesdosis für die Indikation Entzündungshemmung kann unterhalb der ersten Grenzdosis von 1 mg/kg Körpergewicht liegen, oder für die Indikation Immunstimulation oberhalb der zweiten Grenzdosis von 2 mg/kg Körpergewicht.

Eine erfindungsgemäße Wirksubstanz bindet an CD28 oder an eine Teilsequenz hieraus. Die Teilsequenz kann beispielsweise eine Aminosäurensequenz Seq.-ID 1, oder 2 - 7, oder 17 enthalten, welche zumindest teilweise im Bereich des C'-D Loops von CD28 liegen. An eine der Sequenzen mit Val am 5'-Ende kann eine oder mehrere Aminosäuren der Sequenz 8 in der dort definierten Reihenfolge angeschlossen sein. Das Loop liegt im Bereich mit der Sequenz GNYSQQLQVYSKTGF. Erfindungsgemäße Mimikriverbindungen lassen sich in einem Screeningverfahren identifizieren, wobei eine prospektive Mimikriverbindung oder eine Mischung von prospektiven Mimikriverbindungen einem Bindungsassay mit CD28 oder einer Teilsequenz hieraus, insbesondere dem C'-D-Loop, unterworfen werden, und wobei an CD28 oder an die Teilsequenz hieraus bindende Wirksubstanzen selektiert werden, ggf. gefolgt von einem Assay zur Prüfung auf superagonistische Stimulation von mehreren bis allen Untergruppen der T-Lymphozyten. Im Falle einer Mischung wird es zweckmäßig sein, eine Dekonvolution durchzuführen. Unter den selektierten Mimikriverbindungen kann gleichsam ein Ranking nach Maßgabe der Selektivität und/oder Affinität erfolgen, wobei hochaffine Wirksubstanzen bevorzugt sind. Zusätzlich oder anstelle eines solchen Rankings kann ein Ranking anhand einer Quantifizierung der Induktion der Treg erfolgen bzw. anhand der Hemmung der Erkrankung beispielsweise im Tierversuch anhand von Krankheitsmodellen.

Ein Beispiel einer erfindungsgemäß verwendeten Wirksubstanz ist ein superagonistischer CD28 spezifischer mAb. Er ist beispielsweise herstellbar, indem ein nichtmenschliches Säugetier mit CD28 oder einem Peptid mit einer Teilsequenz hieraus, beispielsweise wie vorstehend angegeben oder Homologen hierzu, immunisiert wird, wobei aus dem nichtmenschlichen Säugetier Zellen entnommen und aus den Zellen Hybridomzellen hergestellt werden, und wobei die so erhaltenen Hybridomzellen mit der Maßgabe selektiert werden, daß in deren Kulturüberstand mAb enthalten sind, die an CD28 binden. Mit üblichen Verfahren kann eine Humanisierung durchgeführt werden. Geeignete mAb lassen sich alternativ dadurch herstellen, daß B-Lymphozyten selektiert werden, welche an das Loop binden, und deren exprimierte Immunglobulingene kloniert werden. Auch ist eine Isolierung geeigneter mAb aus Phagenbibliotheken möglich.

Im Einzelnen kann es sich um einen mAb handeln, welcher mit Hybridomzellen, wie hinterlegt unter den DSM Nummern DSM ACC2531 (mAb: 9D7 bzw. 9D7G3H11) oder DSM ACC2530 (mAb: 5.11A bzw. 5.11A1C2H3), erhältlich ist. Der mAb kann eine oder mehrere der Sequenzen Seq.-ID 9, 11, 13 und/oder 15, oder eine oder mehrere Sequenzen Seq.-ID 10, 12, 14, 16, 18 und/oder 19 oder hierzu homologe Sequenzen enthalten bzw. dadurch (teilweise) codiert sein. In SEQ-ID 13 ist die Nukleinsäuresequenz der variablen Region der schweren Kette eines erfindungsgemäßen mAb 5.11A wiedergegeben. SEQ-ID 14 zeigt das hierdurch codierte Peptid. SEQ-ID 15 zeigt die Nukleinsäuresequenz der variable Region der leichten Kette dieses mAb. SEQ-ID 16 ist das hierdurch codierte Peptid. SEQ-ID 9 zeigt die Nukleinsäuresequenz der variablen Region der leichten Kette eines erfindungsgemäßen mAb 9D7 wiedergegeben. SEQ-ID 10 zeigt das hierdurch codierte Peptid. SEQ-ID 11 zeigt die Nukleinsäuresequenz der variable Region der schweren Kette dieses mAb. SEQ-ID 12 ist das hierdurch codierte Peptid. SEQ-ID 18 und 19 zeigen Aminosäurensequenzen der variablen Region eines humanisierten mAb 5.11A, und zwar der leichten Kette und der schweren Kette, respektive.

Die Erfindung betrifft schließlich auch Heilverfahren, bei welchen einer auf niedrige Regulator T-Zellzahlen bzw. hohe T-Lyphozyten-Infiltration in Organen oder Gewebe beruhenden Erkrankung, beispielsweise einer an Guillain-Barre-Syndrom (GBS) und/oder chronischer demyelinisierender Polyneuropathie (CDP) leidenden Person, eine erfindungsgemäße pharmazeutische Zusammensetzung in pharmakologisch wirksamer Dosierung und in für die Verabreichung geeigneter galenischer Herrichtung verabreicht wird.

### Definitionen.

Als monoklonale Antikörper (mAb) sind Antikörper bezeichnet, die von Hybrid-Zellinien (sog. Hybridomen) produziert werden, die typischerweise durch Fusion einer Antikörper produzierenden B-Zelle tierischer oder menschlicher Herkunft mit einer geeigneten Myelom Tumorzelle entstanden sind.

Die Aminosäuresequenz von Human CD28 ist unter der Accession No. NM_006139 bekannt.

Das C'-D Loop von CD28 umfaßt die Aminosäuren 52 bis 66 der vorstehenden CD28 Sequenz (zur Numerierung siehe auch Ostrov, D.A., et al.; Science (2000), 290:816-819). Unter dem Begriff des C'-D Loops sollen im Folgenden auch beliebige Teilsequenzen hieraus verstanden sein.

Ein Loop bzw. eine darin angeordnete Bindungsstelle ist frei zugänglich, wenn für einen definierten Bindungspartner für die Bindungsstelle im Loop keine sterische Hinderung durch an das Loop anschließende Sequenzen oder Moleküle vorliegt.

Regulatorische T-Zellen sind CD4+ T-Zellen, welche in Mischung mit naiven CD4+ T-Zellen deren Aktivierung inhibieren. Hierzu gehören insbesondere CD4+CD25+ T-Zellen. Ein weiteres Merkmal regulatorischer T-Zellen ist eine vergleichsweise zu anderen T-Zellen niedrige Expression der hochmolekularen Isoformen von CD45 (human: RA). Für regulatorische T-Zellen ist die konstitutive Expression von CD152 typisch. CD4+CD8-SP Thymocyten sind eine der wesentlichen Quellen für regulatorische T-Zellen. Für eine weitergehende Charakterisierung von regulatorischen T-Zellen wird auf die Literaturstelle K.J. Maloy et al., Nature Immunology, Vol. 2, No. 9, Seiten 816 ff., 2001, verwiesen.

Mit Induktion regulatorischer T-Zellen ist die Vermehrung der Stoffwechselaktivität, Vergrößerung des Zellvolumens, Synthese immunologisch wichtiger Moleküle und Eintritt in die Zellteilung (Proliferation) auf einen äußeren Reiz hin bezeichnet. Im Ergebnis liegen nach der Induktion mehr regulatorische T-Zellen vor als vorher.

Homologie bezeichnet eine zumindest 70%ige, vorzugsweise zumindest 80%ige, höchstvorzugsweise zumindest 90%ige Sequenzidentität auf Proteinebene, wobei ein homologes Protein oder Peptid einen definierten Bindungspartner mit zumindest gleicher Affinität bindet. Abweichungen in der Sequenz können Deletionen, Substitutionen, Insertionen und Elongationen sein.

Eine Mimikriverbindung ist eine natürliche oder synthetische chemische Struktur, die sich in einem definierten Bindungsassay wie ein definierter mAb verhält, welchen die Mimikriverbindung mimikriert.

Der Begriff der mAb umfaßt neben Strukturen des üblichen Fab/Fc Aufbaus auch Strukturen, die ausschließlich aus dem Fab-Fragment bestehen. Es ist auch möglich, lediglich die variable Region zu nutzen, wobei das Fragment der schweren Ketten mit dem Fragment der leichten Kette auf geeignete Weise, beispielsweise auch mittels synthetischer Brückenmoleküle, verbunden ist. Der Begriff der Antikörper umfaßt auch (ggf. vollständige) chimäre sowie humanisierte Antikörper.

Superagonistische Stimulation der Proliferation von CD28 spezifischen T-Zellen meint, dass keine Costimulation, i.e. kein weiteres Bindungsereignis neben einer Bindung eines mAb oder einer Mimikriverbindung an CD28 zur Stimulation oder Inhibierung der Proliferation erforderlich ist, und dass mehrere bis alle Untergruppen der T-Lymphozyten von solchen superagonistischen CD28 spezifischen mAb aktiviert werden.

Eine untere Grenzdosis läßt sich für jeden erfindungsgemäß eingesetzten Wirkstoff dadurch individuell ermitteln, dass zunächst die Zellzahlen von CD25+ und CD25- Zellen pro Volumeneinheit in dem Blut eines Organismus bestimmt werden, dass dann dem Organismus oder einem gleichen Organismus mit jeweils steigender Dosis der Wirkstoff verabreicht wird, wobei beispielsweise 3 bis 20 Tage nach der Gabe einer Dosis wiederum die Zellzahlen von CD4+CD25+ und CD4+CD25- Zellen pro Volumeneinheit Blut bestimmt werden, wobei in einem Diagramm Zellzahlen (Ordinate) gegen Dosis (Abzisse) die erhaltenen Werte eingetragen werden, wobei die Werte zu Kurven verbunden werden, beispielsweise durch einen Polynomfit, wobei die erste Ableitung der Kurven nach der Dosis gebildet wird und wobei die untere Grenzdosis als jene Dosis ermittelt wird, bei welcher die erste Ableitung der Kurve für CD4+CD25+ den gleichen Wert, wie die erste Ableitung der Kurve für CD4+CD25- aufweist, und zwar innerhalb einer Toleranzabweichung von weniger als 50% (bezogen auf den Wert der Ableitung für CD4+CD25+), vorzugsweise weniger als 30%, höchstvorzugsweise weniger als 10%. Dieser Wert kann gleichzeitig auch die zweite Grenzdosis sein. Es kann aber auch vorgesehen sein, dass die untere Grenzdosis jener Wert ist, bei welchem der Wert der Ableitung für CD4+CD25- das Vorzeichen von negativ nach positiv wechselt. Der obere Grenzwert kann dann wie bereits vorstehend angegeben bestimmt sein. Alternativ kann die untere Grenzdosis dadurch definiert sein, dass die relative Zunahme in besagten Zeitraum der CD4+CD25+ Zellen im Organismus mindestens um einen Faktor zwei höher ist als die relative Zunahme der CD4+CD25- Zellen (Beispiel: Vervierfachung der CD4+CD25+ Zellen bei Verdoppelung der CD4-CD25- Zellen. Die obere Grenzdosis könnte dann als gleiche relative Zunahme beider Zelltypen definiert sein. Für den Menschen werden typische Werte für die untere Grenzdosis bei weniger als 1 mg/kg Körpergewicht und Tag liegen. Die obere Grenzdosis wird bei 2 mg/kg Körpergewicht und Tag liegen.

Typische Erkrankungen der Indikation Entzündungshemmung sind rheumatoide Arthritis, multiple Sklerose, insulinabhängiger (Typ 1) Diabetes Mellitus, Morbus Crohn, Psoriasis, Guillain-Barre-Syndrom (GBS), Graft-versus-Host-Disease (GvHD).

Typische Erkrankungen der Indikation Immunstimulierung sind chronische lymphozytäre Leukämie des B Zell-Typs (B-CLL), akute lymphoblastische Leukämie (A-LL), T-Lymphopenie nach Chemo- oder Strahlentherapie, wie sie beispielsweise bei diversen soliden Tumoren, wie Lungenkarzinom oder Mammakarzinom, erfolgt, HIV Infektion, HTLV Infektion.

### Ausführungsbeispiele.

Im Folgenden werden die Erfindung sowie die zu Grunde liegende Erkenntnisse näher erläutert.

FIG. 1 zeigt die Langzeit-Expansion regulatorischer CD4+CD25+ T-Zellen in vitro. Von Lin et al. (Lin and Hunig, 2003) wurde gezeigt, dass der superagonistische anti-Ratten-CD28 mAb JJ316 in der Lage ist, regulatorische CD4+CD25+CTLA-4+ T-Zellen in Kurzzeit-Zellkultur sehr effizient zu aktivieren. Es stellte sich die Frage, ob es auch zu einer lang anhaltenden Expansion von Treg kommt. FIG. 1a zeigt, dass dieser Antikörper auch eine langfristige Expansion der CD4+CD25+ T-Zellen ermöglicht: 6x10^4 CD4+CD25+ bzw. CD4+CD25- T-Zellen wurden aus einer normalen Lewis-Ratte isoliert und in Anwesenheit von JJ316 (5 µg/ml) und Interleukin-2 (300 U/ml) in Mikrotiterplatten (96-Napf, 12-Napf oder 6-Napf-Platten) bei 37°C kultiviert, wobei die Zellen wöchentlich mit frischen Reagenzien versorgt wurden. Auch in dieser Langzeit-Kultur ist eine präferentielle Expansion der CD4+CD25+ Zellen gegenüber den CD4+CD25- Zellen zu beobachten. Ausgehend von der Ausgangszellzahl (FIG. 1a zeigt relative Werte bezügl. der Zellzahl; die absolute Zellzahl am Tag 0 wurde gleich 1 gesetzt) konnten die CD4+CD25+ T-Zellen um den Faktor 6,1x10^7 vermehrt werden. Bei den CD4+CD25- Zellen beträgt dieser Wert lediglich 6,7x10^5 (FIG. 1a). Die funktionellen Eigenschaften von regulatorischen T-Zellen in vitro sind dadurch charakterisiert, dass sie die Proliferation konventioneller T-Zellen unterdrücken können. Mittels eines *in vitro* Testsystems wurde untersucht, ob die durch JJ316 expandierten CD4+CD25+ T-Zellen diese Funktion beibehalten konnten (FIG. 1b): 5 x 10^4 konventionelle CD4+ T-Zellen, die bei diesem Test als Indikatorzellen dienten, wurden entweder alleine oder zusammen mit ansteigenden Mengen (5x10^3, 1X10^4 oder 5X10^4 Zellen ) der durch JJ316 expandierten CD4+CD25+ bzw. CD4+CD25-TZellen von Tag 78 der Langzeitkultur (FIG.1a) für drei Tage in 96-Napf Mikrotiterplatten in 0,2 ml kultiviert. Die Stimulation der Zellen erfolgte durch Zugabe des Lektins ConA (2 µg/ml) und antigenpräsentierender Zellen (5x10^4). Die Zellproliferation in den Kulturen mittels Einbau von 3H-Thymidin während der letzten 16 Stunden des Assays gemessen, wie in der Literatur beschrieben. Die polyklonale Stimulation der CD4+ Indikatorzellen durch ConA (Säule 1) konnte durch Zugabe der expandierten CD4+CD25+ T-Zellen signifikant reduziert werden (Säulen 2-4). Hingegen zeigten die expandierten CD4+CD25- T-Zellen (Säule 5) wie erwartet keine suppressiven Eigenschaften. Im Gegensatz zu den CD4+CD25- Zellen (Säule 7) konnten die JJ316-stimulierten CD4+CD25+ Zellen (Säule 6) durch ConA nur in sehr geringem Ausmass stimuliert werden, was darauf hinweist, dass die in den Säulen 2-4 gezeigten Werte fast ausschliesslich die Proliferation der Indikatorzellen widerspiegeln. Dieses Ergebnis zeigt, dass die in *vitro* expandierten regulatorischen T-Zellen ihre funktionellen - d.h. suppressiven - Eigenschaften bewahren konnten.

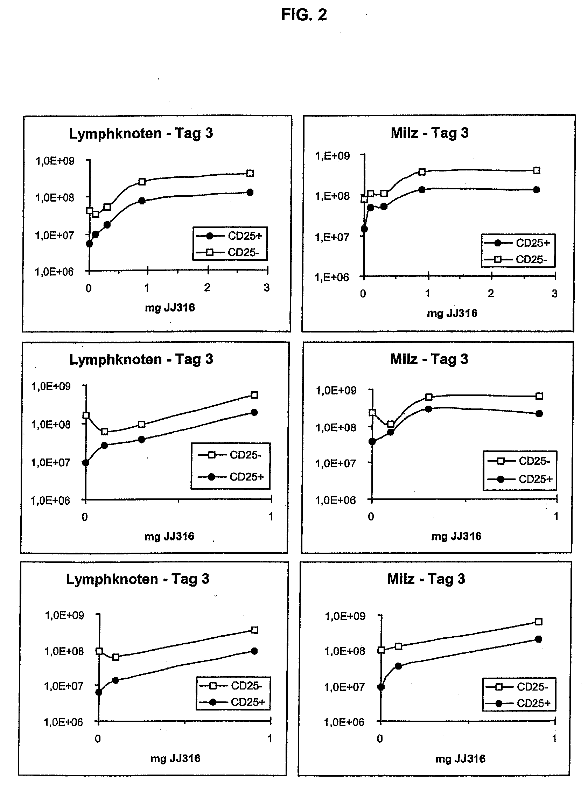

FIG. 2 zeigt die präferenzielle Vermehrung CD4+CD25+ regulatorischer T-Zellen nach *in vivo* Applikation von JJ316 in normalen Lewis-Ratten. Nachdem die oben beschriebenen in vitro-Experimente gezeigt hatten, dass superagonistische CD28-spezifische mAb eine präferentielle Expansion von CD4+CD25+ T-Zellen unter Beibehaltung ihrer funktionellen Eigenschaften induzieren können, wurde untersucht, ob ähnliche Effekte auch nach Injektion dieser Antikörper im Versuchstier zu beobachten sind: Normalen adulten Lewisratten wurde JJ316 in unterschiedlichen Dosen (0,1; 0,3 und 0,9 und 2,7 mg/Tier) intravenös appliziert. Nach drei Tagen wurden Milz und Lymphknoten entnommen und die Lymphozyten Subpopulationen dieser Organe im FACS (gemessene Parameter: CD5, CD4 und CD25) analysiert. Die durch die FACS-Analyse ermittelten relativen Werten der CD4+CD25+ und CD4+CD25- T-Zellen wurden mit der Gesamtzellzahl/Organ multipliziert und die so ermittelte absolute Zahl der beiden Populationen in Abhängigkeit von der applizierten Dosis aufgetragen. In FIG. 2 sind die Ergebnisse dreier unabhängiger Experimente dargestellt. Wie die Steilheit des Kurvenverlaufs speziell im unteren Dosisbereich und besonders in den Lymphknoten zeigt, ist nach in vivo-Applikation von JJ316 eine verstärkte Expansion der CD4+CD25+ im Vergleich zu den CD4+CD25-T-Zellen zu beobachten. Dieses Experiment zeigt eindeutig, dass superagonisitische CD28-spezifische monoklonale Antikörper auch *in vivo* bereits bei niedriger Dosierung vorrangig regulatorisehe T-Zellen vermehren und somit potentiell die bei vielen Autoimmunerkrankungen gestörte Balance zwischen regulatorischen T-Zellen und autoreaktiven T-Zellen wieder ausgleichen können.

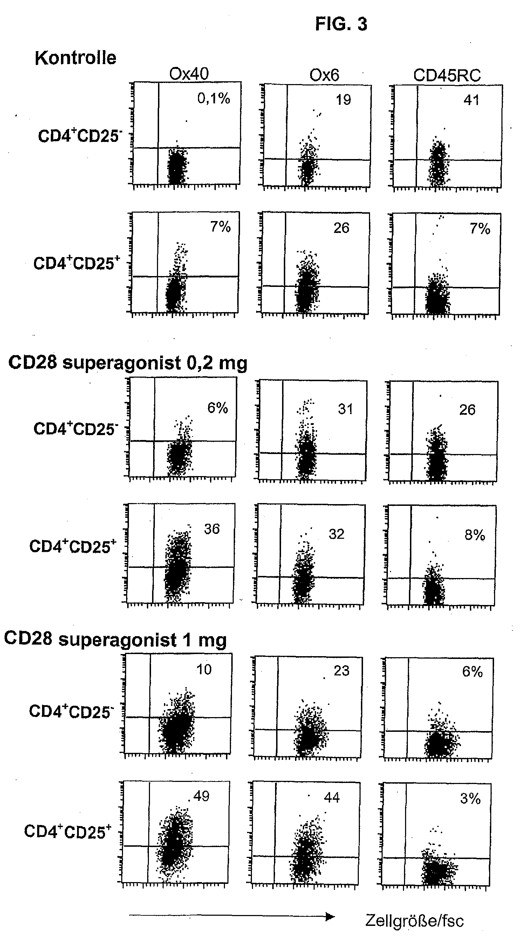

FIG.3 zeigt den Phänotyp JJ316 expandierter Zellen. In dem vorherigen Experiment wurden die absoluten Zellzahlen von CD4+CD25+ und CD4+CD25- T-Zellen nach in vivo Applikation bestimmt und es wurde gezeigt, dass bei der geringeren Dosis JJ316 die Zahl der CD4+CD25+ Zellen im Lymphknoten relativ stärker ansteigt als die Zahl der CD4+CD25-Zellen. Anschließend war es von Interesse, den Aktivierungs- bzw. Differenzierungszustand der CD4+CD25+ und CD4+CD25- T-Zellen mittels durchflusszytometrischer Analyse der Expression der T-Zellmarker OX40, OX6 (MHC Klasse II) und CD45RC zu bestimmen. Männlichen Lewis Ratten wurden 0,2 oder 1 mg JJ316 i.v. injiziert. Vier Tage später, d.h. zu einem Zeitpunkt nahe der maximalen Expansion der CD4+CD25+ Zellen (Lin and Hunig, 2003) wurden Lymphknotenzellen isoliert und es wurde die Zelloberflächenexpression von OX40, OX6 (MHC Klasse II), und CD45RC ermittelt. In FIG. 3 ist die relative Zellgröße (x-Achse) gegenüber den Aktivierungsmarkern (y-Achse) unter den CD4+CD25- bzw. CD4+CD25+ Zellen der Kontrollgruppe (oben), der Niedrigdosisgruppe (Mitte) und der Hochdosisgruppe (unten) dargestellt. Hohe Expression von OX40 bzw. OX6 und niedrige Expression von CD45RC sowie Zunahme der relative Zellgröße im sog. "forward scatter", fsc, kennzeichnet aktivierte Zellen. Demnach wurden sowohl CD4+CD25- als auch CD4+CD25+ Zellen durch beide Dosen von JJ316 aktiviert. Unter den konventionellen CD4+CD25- Zellen schien die Aktivierung bei der höheren Dosis stärker auszufallen als bei der bei der niedrigen Dosis (Anstieg insbesondere des fsc und Reduktion von CD45RC, allerdings geringerer Anstieg von OX6). Die regulatorischen CD4+CD25+ Zellen wiesen schon in der Kontrollgruppe erwartungsgemäß einen hohen Anteil von voraktivierten Zellen auf (niedriger Anteil von CD45RC+ Zellen), der durch beide Dosen noch verstärkt wurde (siehe insbesondere fsc, OX40). Die höhere Expression von OX40 und der größere fsc deuten darauf hin, das die Aktivierung in der Hochdosisgruppe stärker ausfiel als in der Niedrigdosisgruppe.

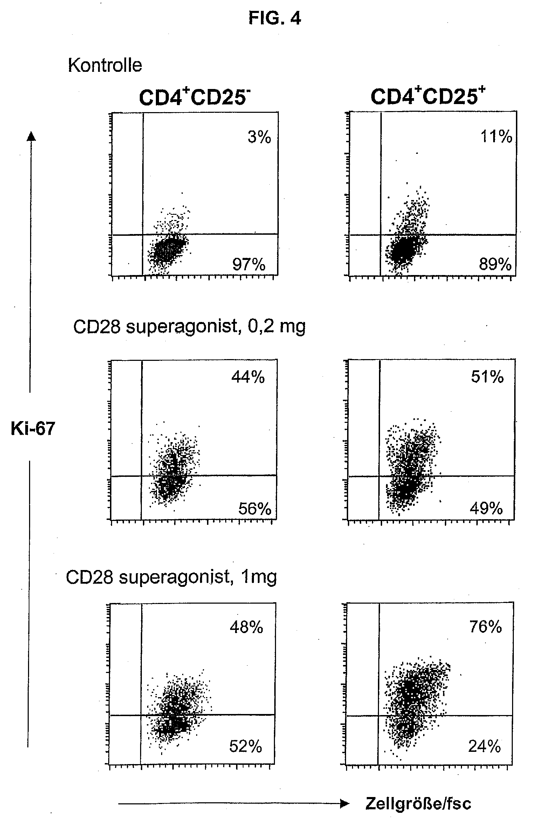

FIG. 4 zeigt in vivo Proliferation von CD4+CD25+ und CD4+CD25- T-Zellen nach Gabe von JJ316. Fig. 3 zeigte, dass sowohl konventionelle CD4+CD25- Zellen als auch regulatorische CD4+CD25+ Zellen in vivo durch beide Dosen von JJ316 phänotypisch aktiviert werden. Anschließend wurde mit Hilfe des Proliferationsmarkers Ki67 gemessen, ob die Aktivierung auch mit einer Zellteilung korreliert. Der Versuchsansatz war identisch wie bei Fig. 3. Der Anteil der sich im Zellzyklus befindenden CD4+CD25+ bzw.

CD4+CD25- Zellen wurde durch extrazelluläre Färbung auf CD4 und CD25 Expression sowie durch intrazelluläre Färbung auf Ki-67 Expression (kennzeichnet Zellen, die sich in S oder G2/M-Phase der Zellteilung befinden und damit aktiv proliferieren), bestimmt. In FIG. 4 ist zu sehen, dass gegenüber der Kontrollgruppe die Behandlung mit JJ316 in beiden Populationen eine starke Ki-67 Expression und damit aktive DNA Synthese induziert, die bei der hohen Dosis geringfügig stärker ausfiel als bei der niedrigen Dosis. Zusammenfassend zeigen die Experimente, dass durch i.v. Applikation von 0,2 mg bzw. 1 mg JJ316 eine profunde phänotypische Aktivierung und Vermehrung von Treg in Ratten induziert wird.

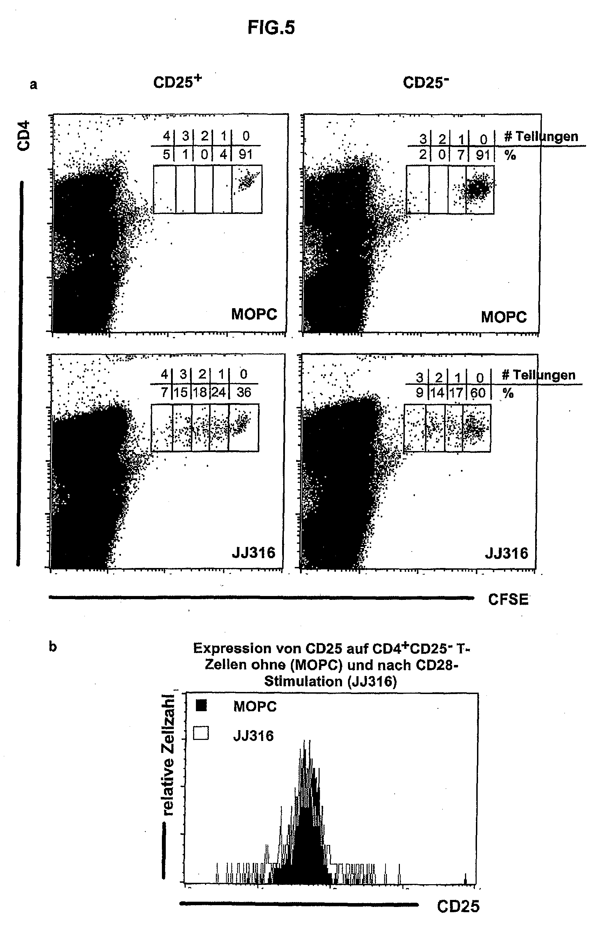

FIG. 5 zeigt, dass JJ316 CD4+CD25+ T-Zellen in vivo expandiert, ohne die Expression von CD25 auf vorher CD25 negativen (konventionellen) T-Zellen zu induzieren. Die bisherigen Experimente bleiben den direkten Beweis schuldig, dass die gesteigerte absolute Zahl der CD4+CD25+ T-Zellen nach in vivo-Applikation von JJ316 die Folge der Expansion dieser Zellen ist und nicht darauf beruht, dass ursprünglich CD25 negative T-Zellen nach Aktivierung dieses Protein als "Aktivierungsmarker" an der Oberfäche exprimieren (wie dies nach in vitro-Aktivierung von T-Zellen zu beobachten ist). Zur Klärung dieser Frage wurden CD4+CD25+/CD4+CD25- T-Zellen aus Milz und Lymphknoten von normalen Ratten isoliert und die beiden Populationen mit dem Farbstoff Carboxyfluorescein Succinylesther (CFSE, 2,5 µM) markiert. CFSE wird in das Zytopiasma der Zelle eingebaut und nach jeder Zellteilung zu je 50% auf die Tochterzellen verteilt. Mittels FACS-Analyse kann der CFSE-Gehalt einer Zellpopulation analysiert und somit die Anzahl der erfolgten Zellteilungen ermittelt werden. 5X10^6 CD4+CD25- bzw. 1X10^6 CD4+CD25+ CFSE-markierte T-Zellen wurden in jeweils unterschiedliche Empfängertiere transferiert, denen einen Tag später entweder ein Kontrollantikörper (MOPC) oder JJ316 jeweils in der niedrigen Dosis von 0,2 mg/Tier intravenös appliziert wurde. Nach zwei Tagen wurden Milz und Lymphknoten dieser Tiere entnommen, die T-Zellen mittels Nylonwolle gereinigt und ihr CFSE-Gehalt im FACS analysiert. FIG. 5a zeigt, dass die Applikation von JJ316 nicht nur eine Verdünnung des CFSE-Gehaltes der transferierten CD4+CD25- sondern auch der CD4+CD25+ T-Zellen induziert. Dieses Ergebnis liefert somit den direkten Beweis, dass JJ316 *in vivo* eine Expansion von CD4+CD25+ T-Zellen induzieren kann. Auch in diesem Experiment können wir eine präferenzielle Expansion der CD4+CD25+ T-Zellen durch JJ316 beobachten, da die durchschnittliche Zahl der Zellteilungen bei den CD4+CD25+ T-Zellen höher liegt (1,3) als bei den CD4+CD25- T-Zellen (0,7). Der Befund, dass CD25 in CD4+CD25- T-Zellen durch JJ316-Stimulation *in vivo* nicht induziert wird (FIG. 5b), weist zudem daraufhin, dass die gesteigerte Zahl der CD4+CD25+ T-Zellen ausschließlich die Folge der Expansion von CD4+CD25+ "Mutterzellen" ist.

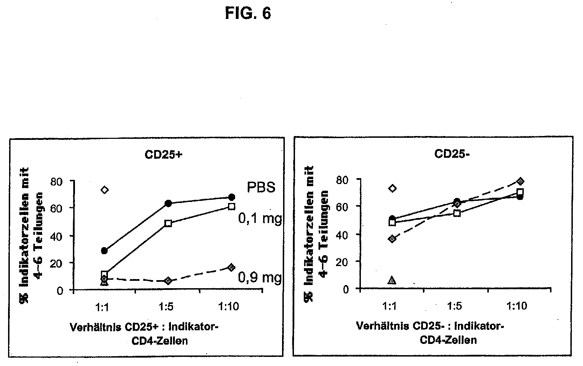

FIG. 6 zeigt die funktionelle Charakterisierung von durch JJ316 *in vivo* expandierten regulatorischen CD4+CD25+ T-Zellen. Von entscheidender Bedeutung ist, ob die durch JJ316 *in vivo* expandierten CD4+CD25+ T-Zellen ihre suppressiven Eigenschaften beibehalten. Zur Klärung dieser Frage wurde normalen Ratten wie oben beschrieben JJ316 in unterschiedlichen Dosen (0,1 mg und 0,9 mg) appliziert. Nach drei Tagen wurden aus den Lymphknoten CD4+CD25+/CD4+CD25- T-Zellen isoliert und in vitro auf ihre suppressorische Potenz getestet: Dabei wurde untersucht, inwieweit die beiden Populationen in der Lage sind, *in vitro* die Proliferation von durch konventionelle Kostimulation aktivierten CD4+ T-Zellen (= Indikatorzellen) zu inhibieren. Die expandierten CD4+CD25+ bzw. CD4+CD25- T-Zellen wurden bei diesem Test in unterschiedlichen Verhältnissen (1:1, 1:5 und 1:10) mit CFSE-markierten CD4+ Indikator-Zellen in Anwesenheit monoklonaler Antikörper mit Spezifität für den T-ZellRezeptor (R73; 1 µg/ml) und CD28 (JJ319; 0,5 µg/ml) für fünf Tage kokultiviert und danach der CFSE-Gehalt der Indikator-Zellen (als Maß der vollzogenen Zellteilungen) im FACS analysiert. Während in Abwesenheit der CD4+CD25+ T-Zellen noch ca. 75% der Indikatorzellen 4-6 Zellteilungen durchlaufen konnten (FIG. 6, offene Raute), war dieser Anteil nach Zugabe der expandierten CD4+CD25+ T-Zellen deutlich reduziert, und zwar umso stärker, je höher die vorher in den Tieren applizierte Dosis von JJ 316 war (FIG. 6, geschlosserie Kreise = PBS-Kontrolle, offene Quadrate = 0,1 mg JJ316, geschlossene Rauten = 0,9 mg JJ316). Hingegen war ein solcher Effekt bei den CD4+CD25- T-Zellen nicht zu beobachten. Nach *in vivo* Applikation superagonistischer CD28-spezifischer monoklonaler Antikörper wird somit nicht nur eine rein numerische Vermehrung regulatorischer T-Zellen induziert, vielmehr können die suppressiven Eigenschaften dieser Zellen mit zunehmender Antikörper-Dosis sogar noch gesteigert werden.

FIG. 7 zeigt den präventiven Effekt von JJ316 auf die Ausprägung von Adiuvans-Arthritis. Die vorangegangenen Ergebnisse zeigen, dass JJ316 in vivo und in vitro eine Expansion funktioneller regulatorischer CD4+CD25+ Zellen induziert. Offen war die wichtige Frage, ob diese Expansion auch mit einem entzündungshemmenden Effekt des Antikörpers im Organismus korreliert und, falls dies der Falls ist, ob die Treg ursächlich an der Vermittlung eines solchen Effektes beteiligt sind. Während nachfolgende Ergebnisse die erste Frage eindeutig bejahen, ist die Frage nach dem Mechanismus der Entzündungsprophylaxe und -therapie, wie in FIG. 7-9 gezeigt, durch CD28-Superagonisten noch nicht eindeutig geklärt. Zur Untersuchung des anti-phlogistischen Effekts von JJ316 wurde das Tiermodell der Adjuvans-Arthritis in Lewis Ratten (Barnes et al., 1998) herangezogen. Zur Krankheitsinduktion wurden 6-8 Wochen alte männliche Ratten (200 bis 250 g) mit 0,1 ml abgetöteten *Mycobaterium tuberculosis* (heat-killed) in IFA (Incomplete Freund's Adjuvant, 5mg/ml) an der Schwanzwurzel intradermal injiziert (Tag 0). Um in der ersten Gruppe den prophylaktischen Effekt von JJ316 zu untersuchen wurden jeweils sechs Ratten am Tag 0 und Tage 10 0,2 mg oder 1 mg JJ316 intravenös appliziert. Als Kontrollgruppe dienten sechs Ratten, die am Tag 0 und Tag 10 mit 0 mg, 0,2 mg oder 1 mg irrelevantem Kontrollantikörper gleichen Isotyps (Maus IgG1) behandelt wurden (gezeigt wird hier sowie in FIG. 8 und 9 eine Gruppe, die mit 1 mg Kontrollantikörper behandelt wurde; identische Ergebnisse wurden in den anderen Kontrollgruppen erzielt). Die Entwicklung von Arthritis wurde durch objektive Auswertung der Gelenkenentzündungen, dargestellt als Arthritis Index bestehend aus einer kombinierten Bestimmung der Rötung, Schwellung und Steifheit der Gelenke und dem Surrogatparameter "Gewichtmessung" kontinuierlich untersucht. Das Experiment wurde am Tag 35 durch Tötung der Tiere beendet. Die Hinterpfoten wurden in 10% Formalinlösung für 72 Stunden fixiert. Nach der Entkalkung in 10% EDTA Lösung (24 Wochen) wurden das Gelenkgewebe wie bei (Barnes et al., 1998) beschrieben durch Färbungen mit 0-Safranin, Fast-Green und Hämatoxilin histologisch aufgearbeitet und hinsichtlich Knorpelzerstörung und zellulären lnfiltraten analysiert. In FIG. 7a,b ist das Ergebnis der präventiven Behandlung von jeweils sechs Tieren im Mittelwert zusammengefasst. Daraus geht hervor, dass in den Kontrolltieren eine Arthritis beginnend am Tag 9 mit einem Maximum nach ca. drei Wochen auftritt (oben rechts), die von einem begleitenden Gewichtsverlust gekennzeichnet ist (Mitte rechts). Durch Behandlung mit niedriger Dosis von 0,2 mg JJ316 (linke Spalte) konnte ebenso wie mit einer hohen Dosis von 1 mg JJ316 (mittlere Spalte) die Ausprägung der Arthritis sowie der Gewichtsveriust fast vollständig verhindert werden. Bemerkenswert ist, dass der prophylaktische Effekt der niedrigen Dosis mindestens genauso gut ist wie der Effekt der 5-fach höheren Dosis. Den gleichwertigen Schutz vor der Arthritis durch hoch und niedrig dosierte JJ316 Behandlung konnte auch auf histologischer Ebene gezeigt werden: Während bei den Kontrolltieren die Gelenke der Hinterpfoten durch massive zelluläre Infiltrate als Zeichen einer Entzündungsreaktion gekennzeichnet sind (FIG. 7c, rechts), weisen die Gelenkspalte der JJ316 behandelten Tiere eine normale Architektur auf (FIG. 7c, Mitte und links). Das Behandlungsschema ist in FIG. 7d zusammengefasst. Zusammenfassend zeigen diese Ergebnisse, dass hoch und niedrig dosierte JJ316 Behandlung zum Zeitpunkt der Arthritisinduktion einen gleich starken präventiven Effekt auf die Ausprägung der Arthritis haben.

FIG. 8 zeigt den therapeutischen Effekt von JJ316 Behandlung auf Adjuvans-Arthritis (frühe Intervention). Als nächstes wurde die wichtige Frage untersucht, ob JJ316 Behandlung nicht nur einen präventiven, sondern auch einen kurativen Effekt auf die Krankheitsausprägung besitzt.

Dies ist besonders relevant, da für klinische Behandlung der RA mit dem humanen CD28-SuperMAB keine Prophylaxe, sondern ausschließlich eine Therapie in Frage kommt. In dem in FIG. 8 dargestellten Experiment wurde bei jeweils sechs Tieren / Gruppe Arthritis wie in FIG. 7 beschrieben induziert. Am Tag 12 und 15 nach Krankheitsinduktion, d.h. zum Zeitpunkt einer sehr milden Ausprägung der Symptome (durchschnittlicher Arthritis Index 0,5) sowie drei Tage später wurden die Tiere mit 0,2 mg JJ316, 1,0 mg JJ316 oder einem irrelevanten Kontroll-Antikörper behandelt. Wie in FIG. 8a gezeigt, kam es bei den JJ316 behandelten Tieren nach anfänglicher Ausprägung der Symptome zu einer Stabilisierung der Krankheit (max. Arthritis Index 4-5) und zwischenzeitlich zu einer deutlichen Reduktion der Symptome sowie einer Stabilisierung des Gewichtes. Wiederum war der therapeutische Effekt bei hoher und niedriger Dosis von JJ316 sehr ähnlich. Bei den kontroll-behandelten Tieren kam es hingegen zu einer fulminanten Arthritis mit einem maximalen Arthritisindex von > 12 und einem progressiven Gewichtsverlust (FIG. 8a,b). Ebenso führte die JJ316 Behandlung in beiden Dosen zu einer Erhaltung der normalen Architektur des Gelenkgewebes, das in der Kontroligruppe deutliche Zeichen von entzündlichen Infiltraten und morphologischer Zerstörung aufwies (FIG. 8c). Insgesamt ist festzuhalten, dass bei einer frühen therapeutischen Intervention eine niedrige Dosis von JJ316 ebenso wie eine hohe Dosis von JJ316 die klinische Ausprägung einer schweren Arthritis und die Gewebezerstörung verhindert.

FIG. 9 zeigt den therapeutischen Effekt von JJ316 Behandlung auf Adjuvans-Arthritis (späte Intervention). Anschließend wurde untersucht, ob eine Arthritis-Therapie durch CD28-Superagonist Gabe auch bei einem bereits deutlich ausgeprägten Krankheitsbild möglich ist. Der experimentelle Ansatz entsprach dem in FIG. 8 beschriebenen, allerdings wurden bereits deutlich erkrankte Tiere am Tag 15, d.h. zu einem Zeitpunkt, an dem die Arthritisausprägung durchschnittlich mit 6,5 bewertet wurde, sowie am Tag 18 mit JJ316 (0,2 und 1,0 mg/Tier) bzw. mit einem Kontrollantikörper behandelt (FIG. 9). Bei den JJ316 behandelten Tieren kam zu einer deutlichen Milderung der Krankheitssymptome etwa ab Tag 16. Dieser Effekt war bei niedriger JJ316 Dosis noch ausgeprägter als bei der hohen Dosis. Ebenso wurde das Gewicht bei den Experimentalgruppe stabil während die Kontrollgruppe einen fortschreitenden Gewichtsverlust aufwies (FIG. 8b). Zusammenfassend zeigte auch bei dieser äußerst anspruchsvollen späten pharmakologischen Intervention eine niedrige Dosis von JJ316 eine mindestens ebenso deutliche kurative Wirkung auf den Arthritisverlauf im Rattenmodell wie eine hohe Dosis von JJ316.

FIG. 10 zeigt die präferenzielle Expansion humaner CD4+CD25+ T-Zellen mittels SuperMAB. Wie bereits in früheren Literaturstellen beschrieben, wurden superagonistische monoklonale Antikörper mit Spezifität für humanes CD28 (=SuperMAB) generiert. Auch hier galt es, die Stimulations- und Kulturbedingungen zu optimieren, um eine effiziente Expansion humaner CD4+CD25+ T-Zellen mittels SuperMAB zu gewährleisten: Von freiwilligen gesunden Spendern wurden T-Lymphozyten präpariert und in "konventionelle" CD4+CD25- sowie regulatorische CD4+CD25+ T-Zellen (Reinheit routinemäßig ca. 90%)getrennt. Jeweils 5x10^4 Zellen der beiden Populationen wurden in Anwesenheit von quervernetztem (mittels 40 µg/ml anti-Maus-Ig Antiserum) SuperMAB (10 µg/ml) oder durch klassische Kostimulation mittels quervernetzten monoklonalen Antikörper mit Spezifität für CD3 (10 µg/ml) und CD28 (10µg/ml) in 96-Napf Mikrotiterplatten kultiviert. Nach drei Tagen wurde die Proliferation mittels Einbau von 3H-Thymidin getestet. Ähnlich wie bei JJ316 beschreiben, werden auch durch SuperMAB CD4+CD25+ T-Zellen stärker in die Proliferation getrieben als konventionelle CD4+CD25-T-Zellen (FIG. 10a). Diese Ergebnis wurde in einem Folgeexperiment reproduziert, in dem zusätzlich der Effekt des Wachstumsfaktors Interleukin-2 auf die Stimulation der CD4 Subpopulationen CD4+CD25+ und CD4+CD25- mittels SuperMAB untersucht wurde. Wie in FIG, 10b gezeigt wird, hatte IL-2 in diesem Experiment keinen entscheidenden Einfluss auf die Proliferationsrate von phänotypisch regulatorischen CD4+CD25+ oder konventionellen CD4+CD25- Zellen. Diese in *vitro* Versuche zeigen, dass auch SuperMAB in der Lage ist, vorrangig regulatorische T-Zellen zu vermehren.

FIG. 11 belegt, dass durch SuperMAB in vitro-expandierte CD4+CD25+ Zellen regulatorische T-Zellen sind. Wie bereits im System Ratte erläutert, stellte sich auch hier die Frage, ob die durch SuperMAB expandierten CD4+CD25+ T-Zellen ihre funktionellen Eigenschaften nämlich die Fähigkeit, die Proliferation konventioneller T-Zellen zu unterdrücken beibehalten konnten. Hierzu wurden CD4+CD25+ und CD4+CD25- T-Zellen von gesunden Spendern isoliert und für 12 Tage in Anwesenheit von SuperMAB (0,4 µg/ml) und Interleukin 2 (100 U/ml) kultiviert. Auch in diesem Experiment konnten die CD4+CD25+ T-Zellen durch SuperMAB besser in die Proliferation getrieben werden als CD4+CD25- T-Zellen (FIG. 11a). In einer zweiten Kultur wurden dann die durch SuperMAB expandierten Zellen auf ihre suppressorische Potenz getestet. Analog zu den für die Ratte projektierten Experimenten wurde untersucht, ob sie in der Lage sind, die Proliferation von durch konventionelle Kostimulation aktivierten syngenen und CFSE markierten CD4+ T-Zellen (=Indikatorzellen) zu inhibieren: Die expandierten CD4+CD25+ bzw. CD4+CD25- T-Zellen wurden in unterschiedlichen Verhältnissen (1:1, 1:5 und 1:10) mit CFSE-markierten CD4+ Indikator-Zellen in Anwesenheit monoklonaler Antikörper mit Spezifität für CD3 (0,1 µg/ml) und CD28 (0,05 µg/ml) für fünf Tage kokultiviert und danach der CFSE-Gehalt der IndikatorZellen (als Maß der vollzogenen Zellteilungen) im FACS analysiert. Während in Abwesenheit der CD4+CD25+ T-Zellen noch ca. 30% der Indikatorzellen 3-5 Zellteilungen durchlaufen konnten (FIG. 11b, offene Raute) war dieser Anteil nach Zugabe der SuperMAB-expandierten CD4+CD25+ T-Zellen deutlich reduziert (geschlossene Kreise). Einen geringen suppressorischen Effekt zeigten auch die expandierten CD4+CD25-T-Zellen (offene Quadrate), was evtl. darauf zurückzuführen ist, dass einige wenige CD25+ T-Zellen innerhalb der Population von CD25- T-Zellen vorhanden waren und während der SuperMAB-getriebenen Expansionsphase ihre suppressiven Eigenschaften auf einen Teil der CD25-T-Zellen übertragen haben. Alternativ wäre auch eine Stimulation CD25-regulatorischer T-Zellen durch SuperMAB vorstellbar. Dieser Versuch zeigt, dass - wie bereits im System Ratte gezeigt - auch durch SuperMAB expandierte humane CD4+CD25+ T-Zellen ihre funktionellen Eigenschaften beibehalten.

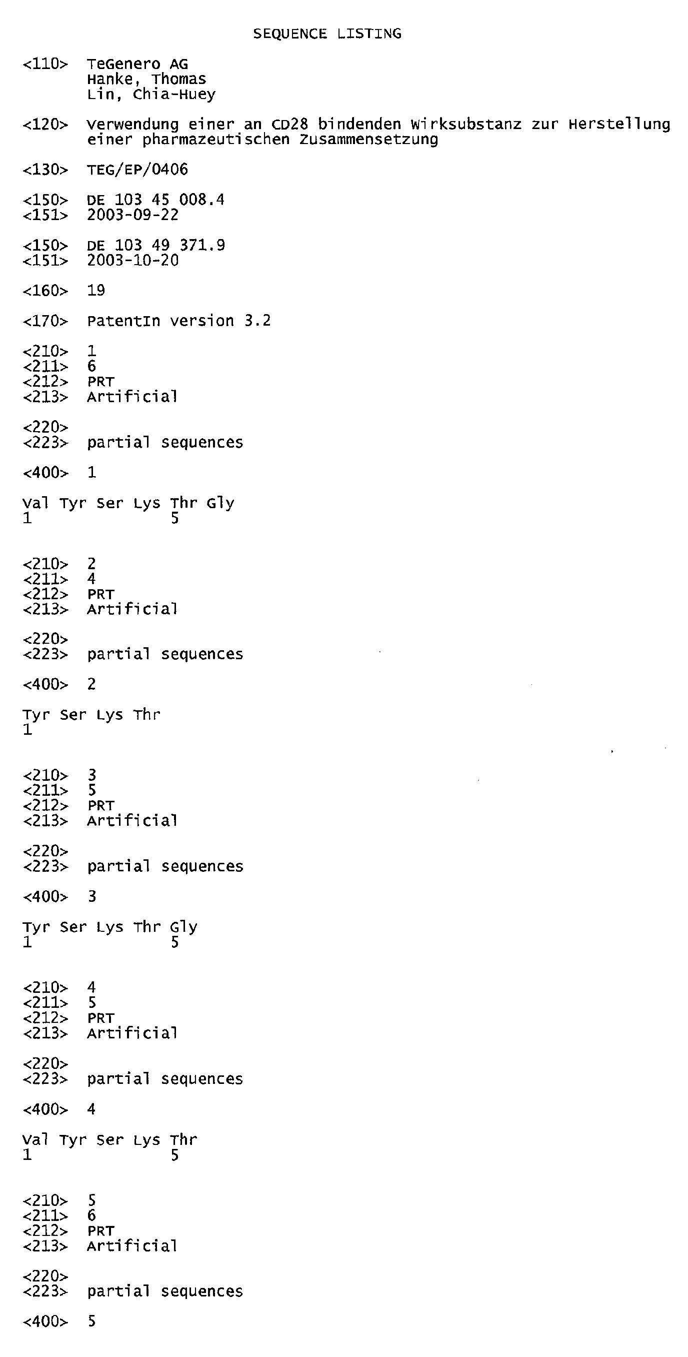

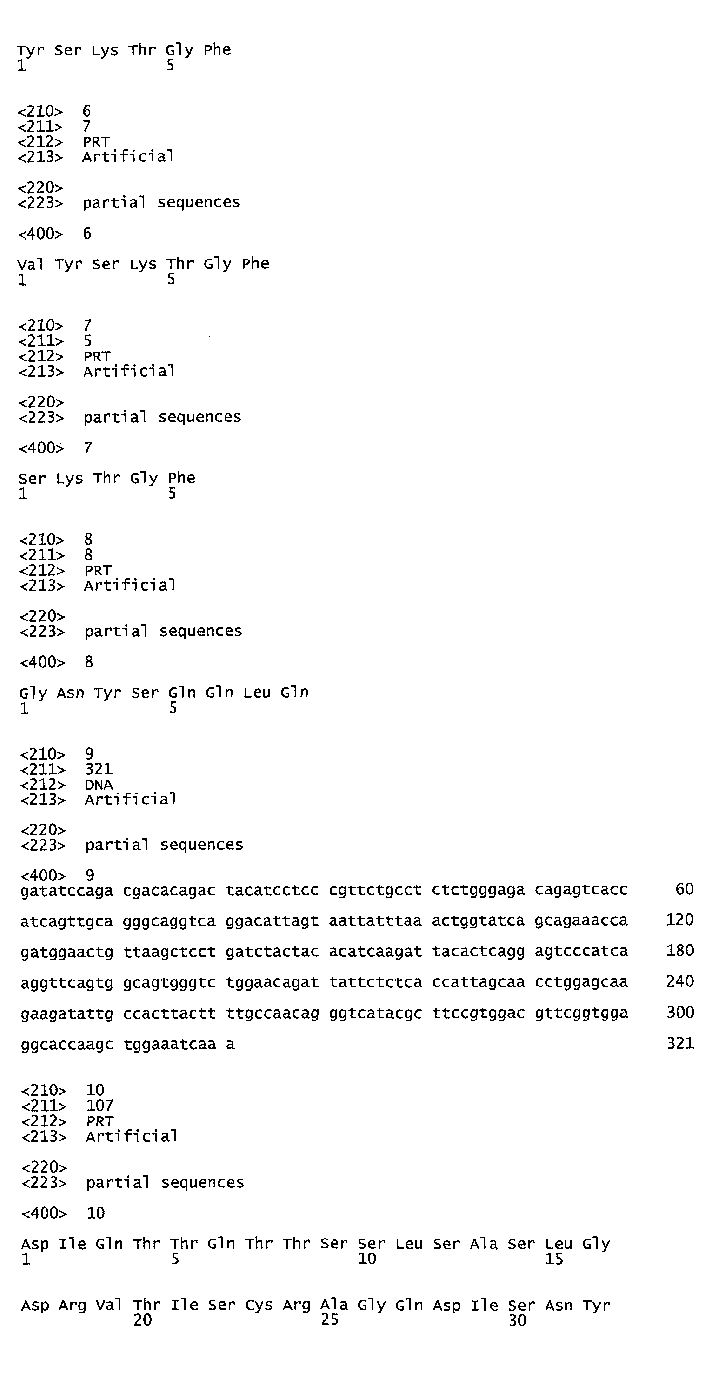

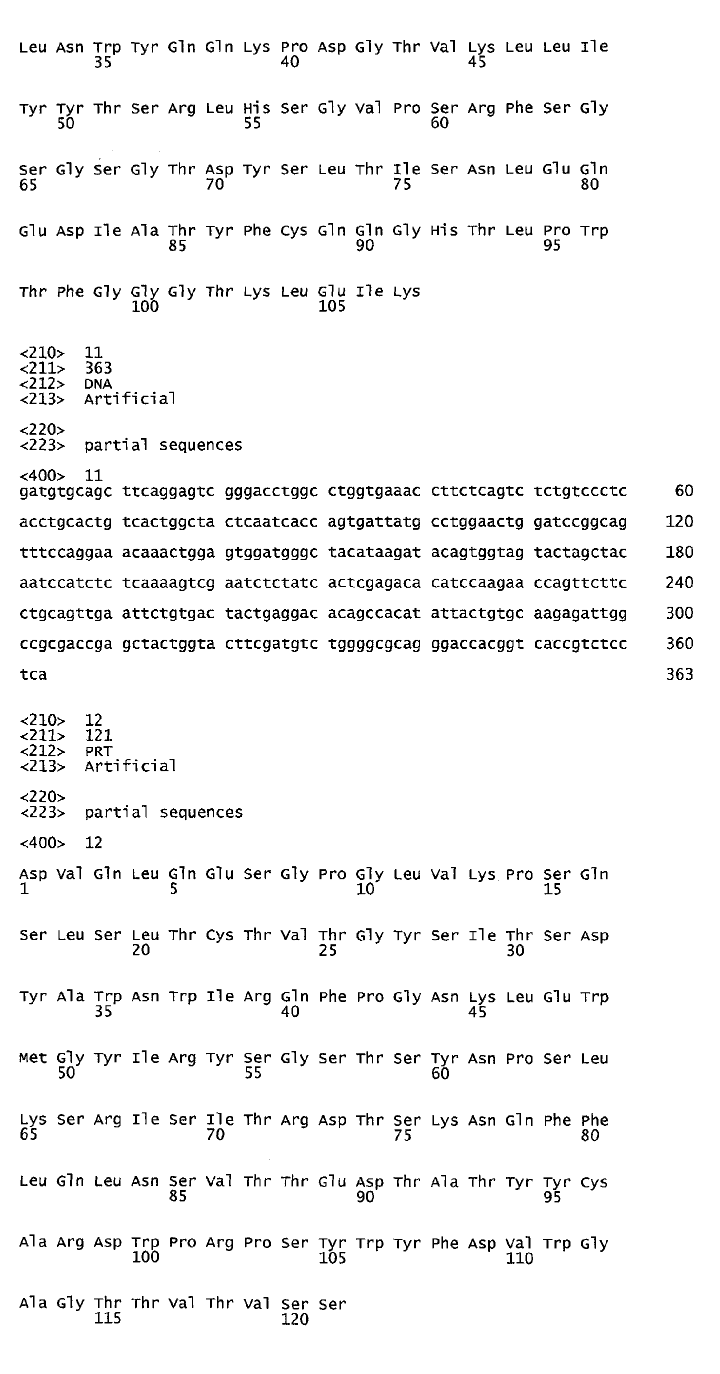

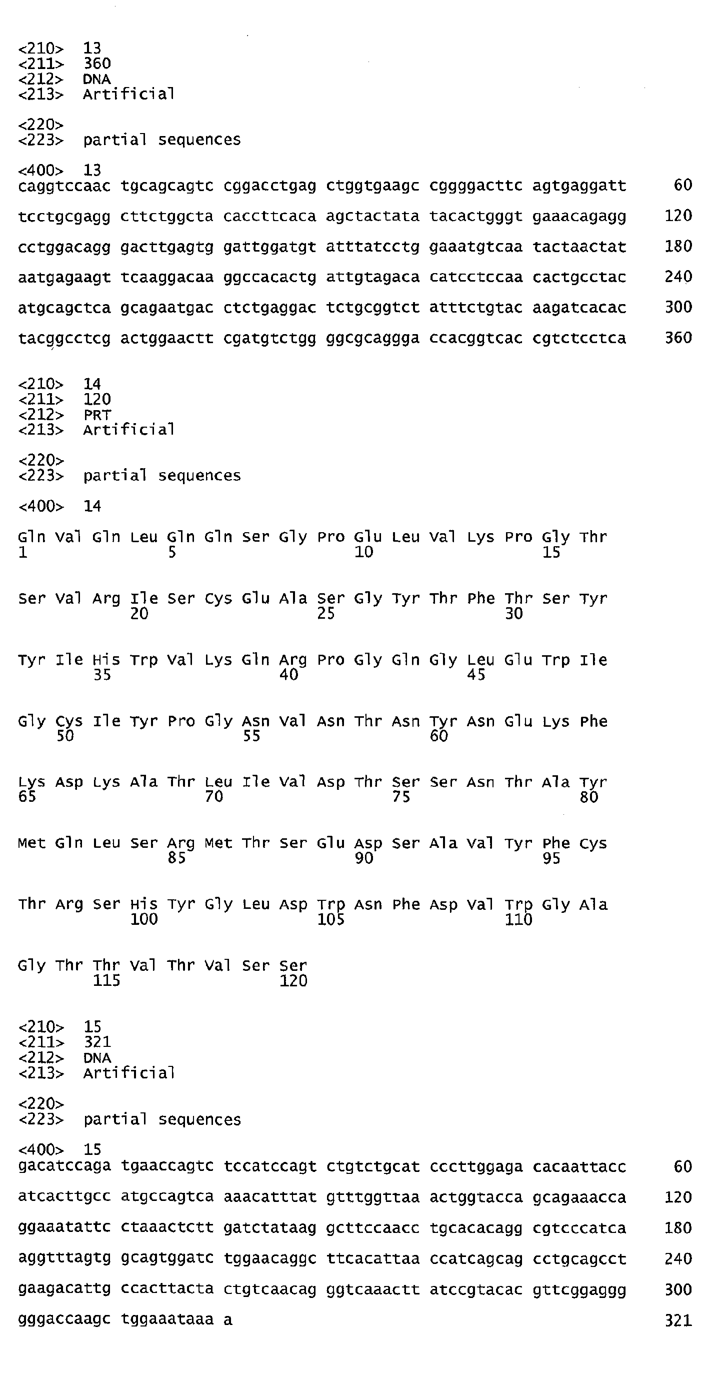

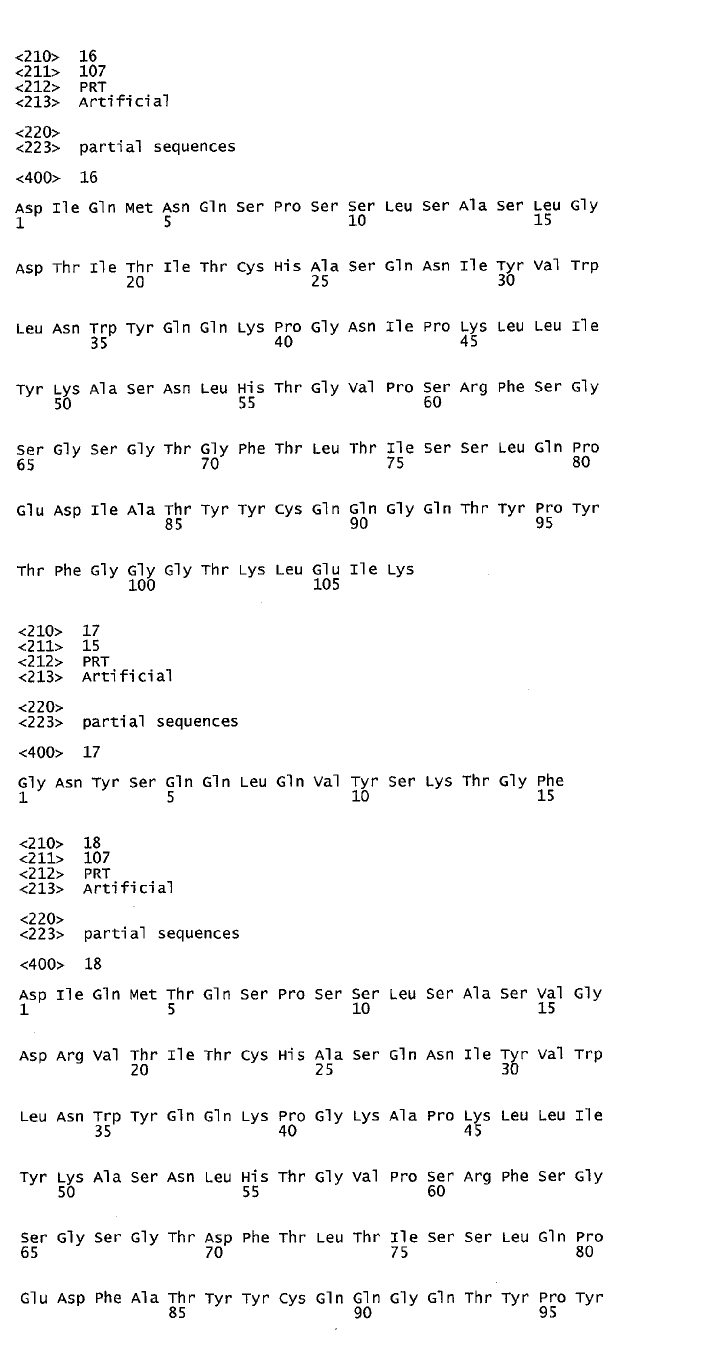

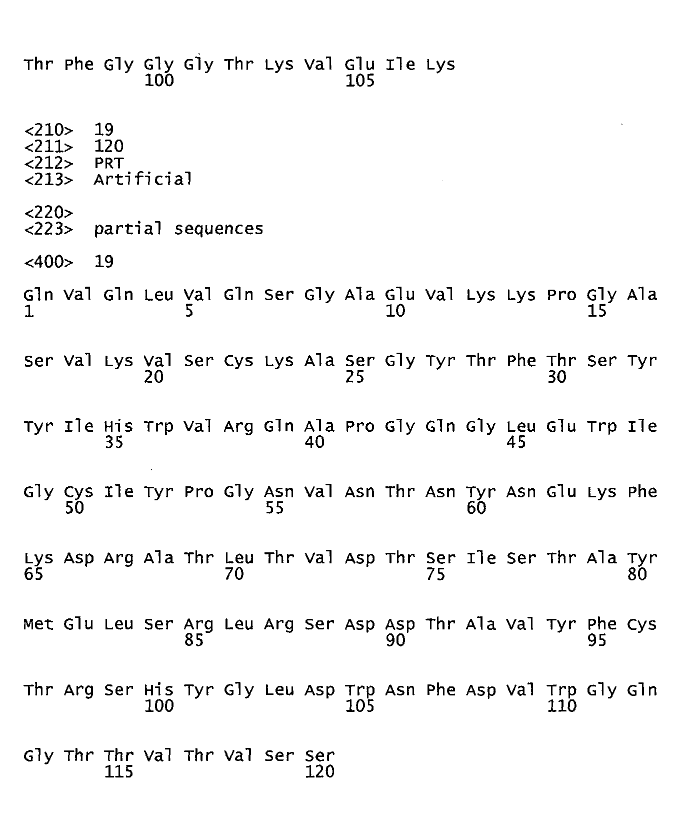

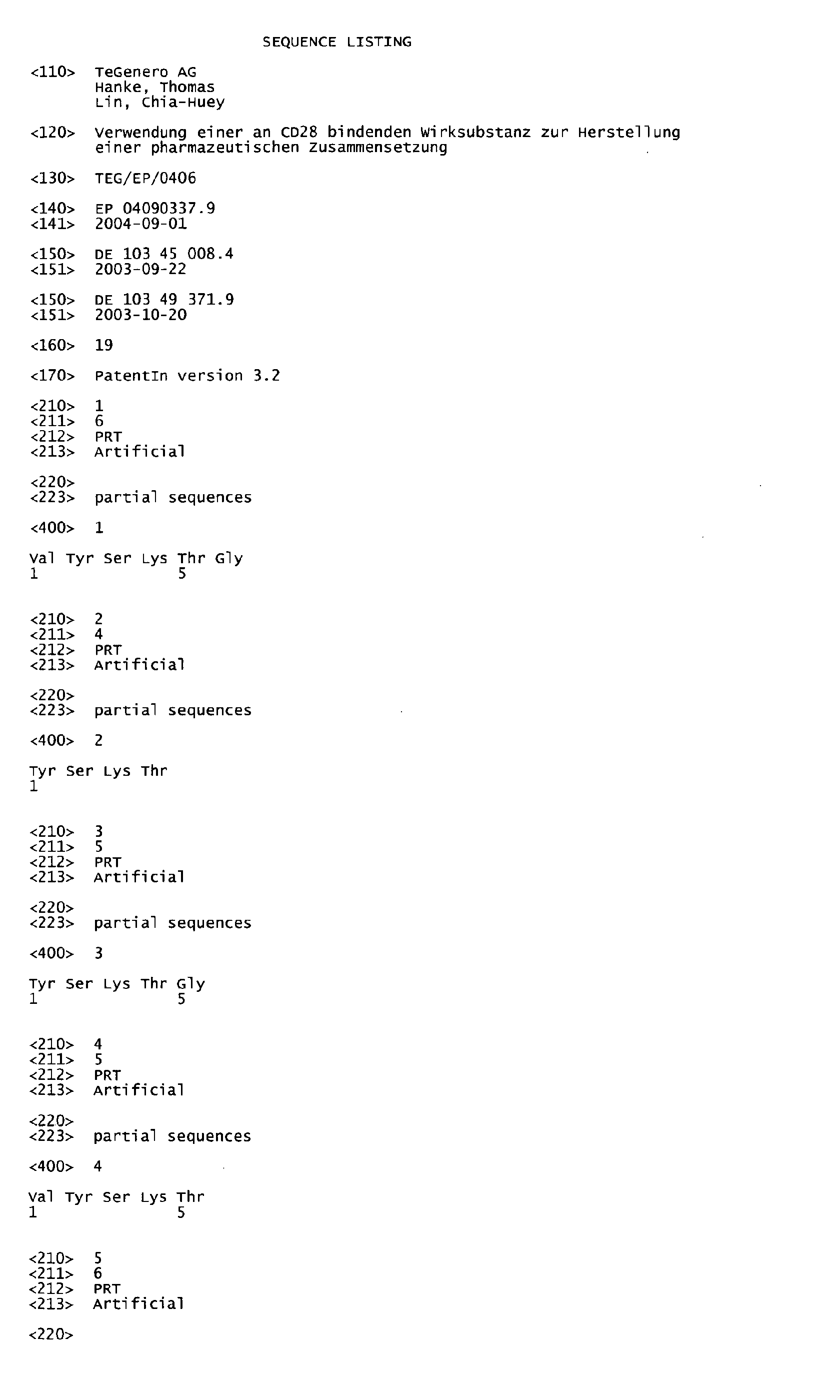

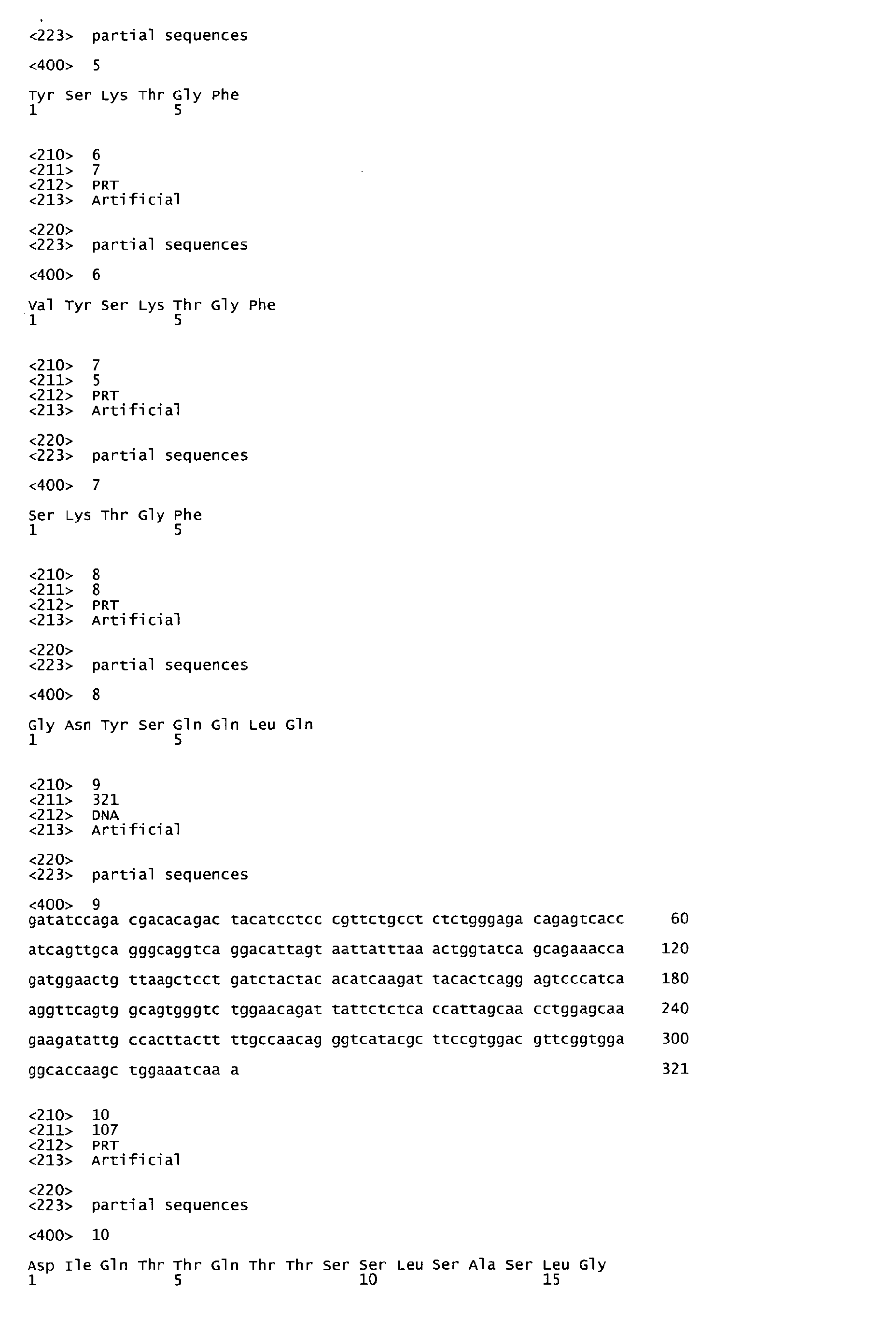

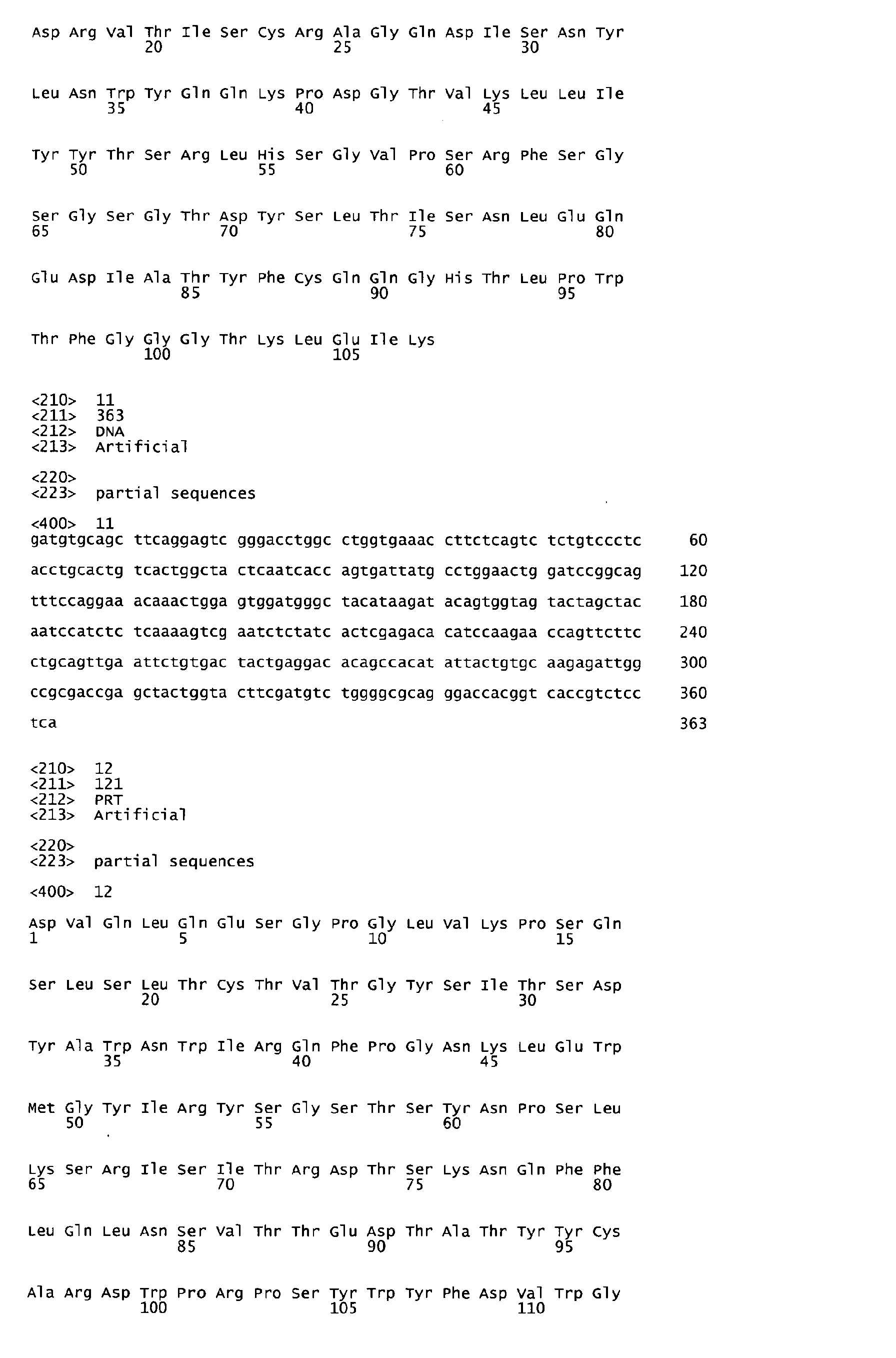

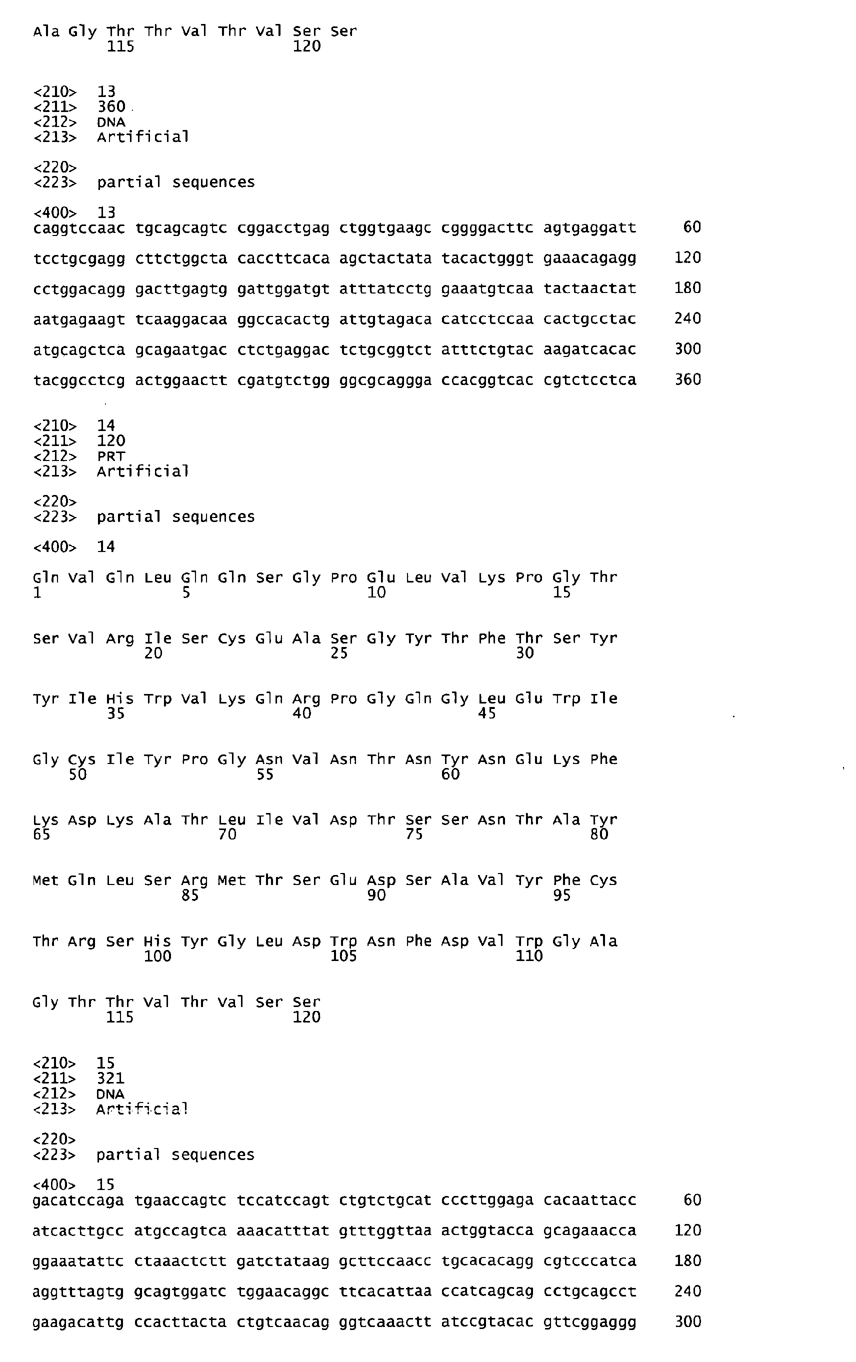

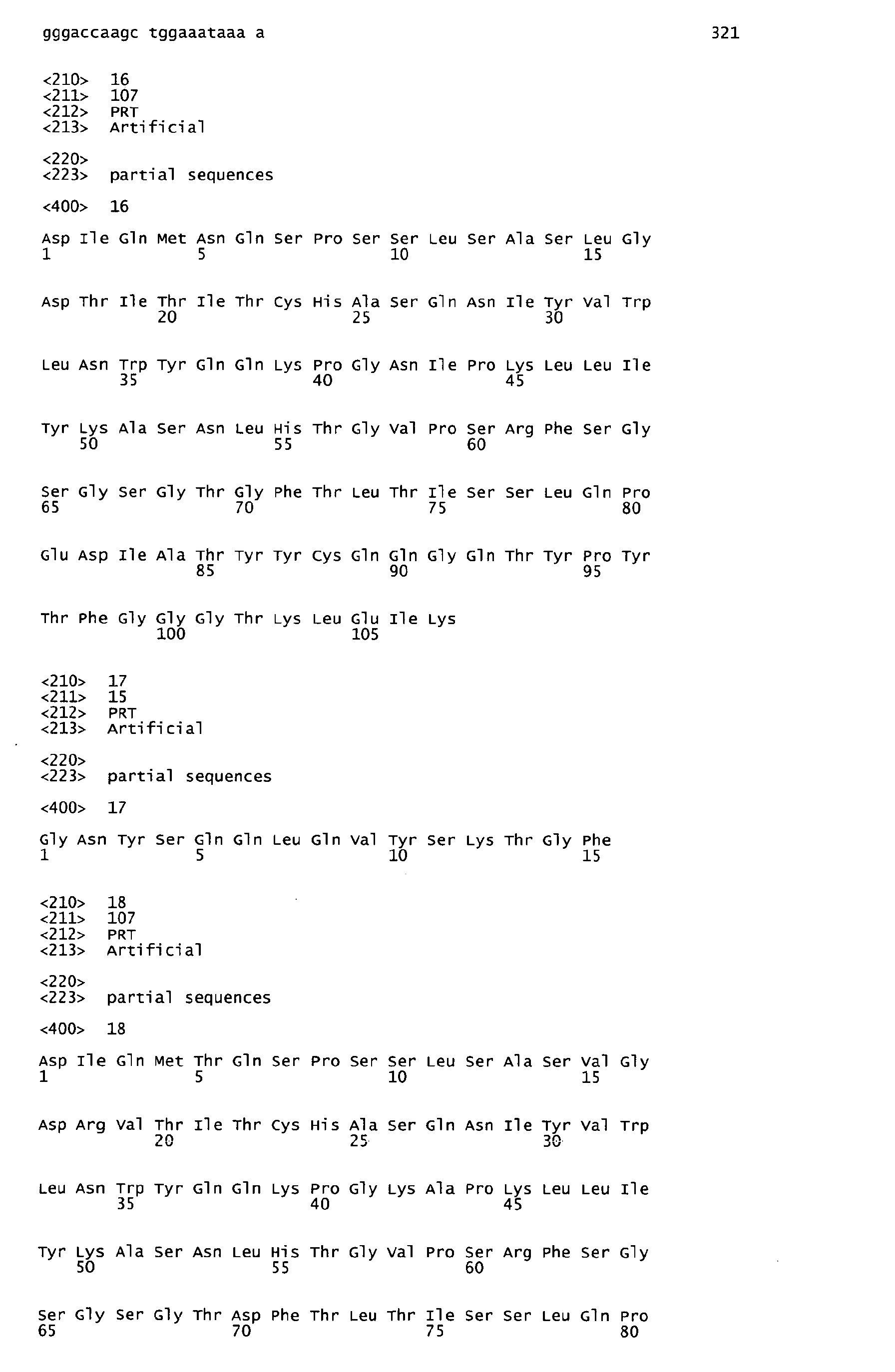

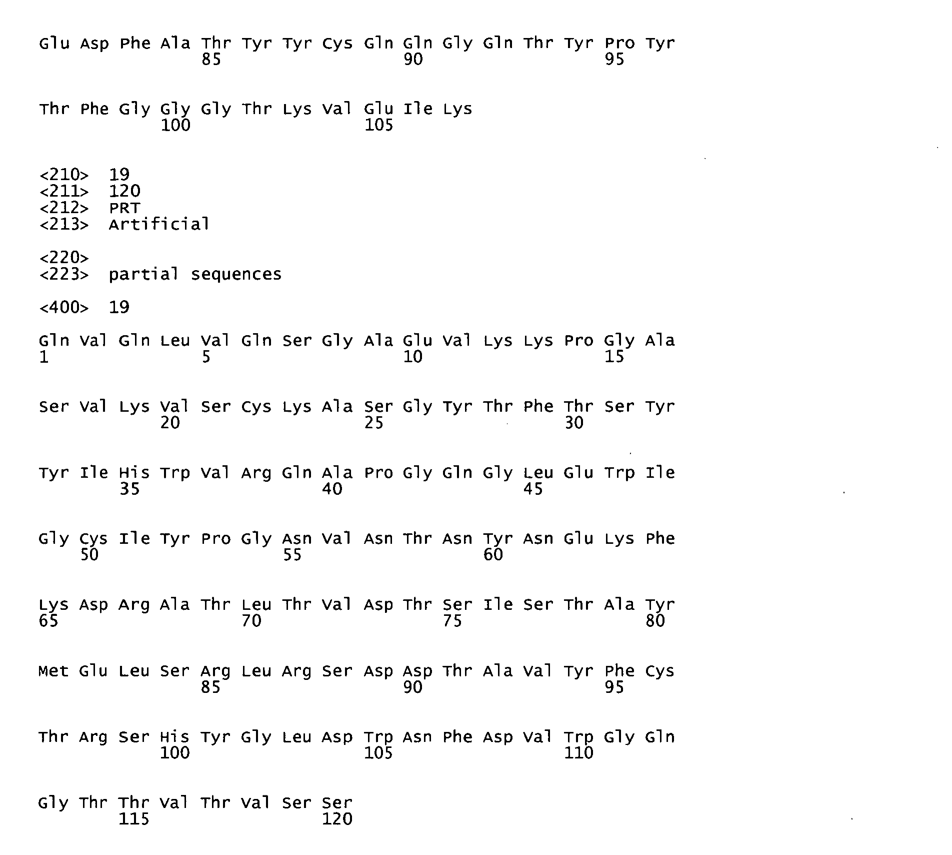

## Patentansprüche

1. Verwendung eines CD28-spezifischen superagonistischen monoklonalen Antikörpers (mAb) oder einer Mimikriverbindung hierzu, zur Herstellung einer pharmazeutischen Zusammensetzung zur Behandlung oder Prophylaxe von autoimmunbedingten entzündlichen Erkrankungen oder zur Immunrekonstitution, wobei die pharmazeutische Zusammensetzung mit der Maßgabe hergerichtet ist, dass die darzureichende Dosis des mAb unterhalb einer definierten ersten Grenzdosis liegt, wenn Behandlung oder Prophylaxe einer autoimmunbedingten entzündlichen Erkrankung indiziert ist, und dass die darzureichende Dosis des mAb oberhalb einer definierten zweiten Grenzdosis liegt, wenn Immunrekonstitution indiziert ist.

2. Verwendung nach Anspruch 1, wobei der mAb herstellbar ist, indem ein nichtmenschliches Säugetier mit CD28 oder einer Teilsequenz hieraus, insbesondere dem C'-D Loop und/oder angrenzenden räumlichen Bereichen, immunisiert wird, wobei aus dem nichtmenschlichen Säugetier Zellen entnommen und aus den Zellen Hybridomzellen hergestellt werden, und wobei die so erhaltenen Hybridomzellen mit der Maßgabe selektiert werden, daß in deren Kulturüberstand mAb enthalten sind, die an CD28 superagonistisch binden.

3. Verwendung nach Anspruch 1, wobei die Mimikriverbindung erhältlich ist in einem Screeningverfahren, wobei eine prospektive Mimikriverbindung oder eine Mischung von prospektiven Mimikriverbindungen einem Bindungsassay mit CD28 oder einer Teilsequenz hieraus, insbesondere dem C'-D-Loop und/oder angrenzenden räumlichen Bereichen, unterworfen werden, und wobei an CD28 oder an die Teilsequenz hieraus bindende Wirksubstanzen selektiert werden, ggf. gefolgt von einem Assay zur Prüfung auf superagonistische Stimulation von mehreren bis allen Untergruppen der T-Lymphozyten, wobei superagonistisch stimulierende Wirksubstanzen selektiert werden.

4. Verwendung nach einem der Ansprüche 1 oder 2, wobei der mAb erhältlich ist aus Hybridomzellen, wie hinterlegt unter den DSM Nummern DSM ACC2531 (mAb: 9D7 bzw. 9D7G3H11) oder DSM ACC2530 (mAb: 5.11A bzw. 5.11A1C2H3).

5. Verwendung nach einem der Ansprüche 1 oder 2, wobei der mAb oder die Mimikriverbindung eine oder mehrere der Sequenzen Seq.-ID 9, 11, 13 und/oder 15, oder eine oder mehrere Sequenzen Seq.-ID 10, 12, 14 und/oder 16, oder eine oder mehrere Sequenzen Seq.-ID 18 und/oder 19 oder 20 und/oder 21, oder hierzu homologe Sequenzen enthält.

6. Verfahren bzw. Behandlungsplan zur Behandlung oder Prophylaxe einer Erkrankung nach Anspruch 1, wobei entweder
einem Patienten eine pharmazeutische Zusammensetzung enthaltend einen CD28-spezifischen superagonistischen monoklonalen Antikörper oder eine Mimikriverbindung hierzu und galenisch hergerichtet für eine definierte und angewandte Darreichungsform, beispielsweise i.v. Injektion, verabreicht wird, oder
einem Patienten eine Körperflüssigkeit, insbesondere Blut, enthaltend T-Lymphozyten oder Vorläuferzellen hierzu entnommen wird, die Körperflüssigkeit, ggf. nach einer Aufbereitungsverfahrensstufe, mit einem CD28-spezifischen superagonistischen monoklonalen Antikörper oder einer Mimikriverbindung hierzu versetzt wird und die so behandelte Körperflüssigkeit dem Patienten wieder dargereicht, beispielsweise i.v. injiziert, wird.

7. Verfahren bzw. Behandlungsplan nach Anspruch 6, wobei die dargereichte Tagesdosis für die Indikation Entzündungshemmung unterhalb der ersten Grenzdosis von 0,1 bis 10 mg/kg Körpergewicht liegt, oder wobei die dargereichte Tagesdosis für die Indikation Immunstimulation oberhalb der zweiten Grenzdosis von 0,1 bis 10 mg/kg Körpergewicht liegt, wobei die zweite Grenzdosis stets gleich der ersten Grenzdosis ist oder höher.
